(19) Europäisches Patentamt
European Patent Office
Office européen des brevets

(11) EP 3 682 900 A1

(12) **EUROPEAN PATENT APPLICATION**
published in accordance with Art. 153(4) EPC

(43) Date of publication:
**22.07.2020 Bulletin 2020/30**

(21) Application number: **18855561.9**

(22) Date of filing: **14.09.2018**

(51) Int Cl.:
***A61K 45/00*** *(2006.01)* ***A61K 31/395*** *(2006.01)*
***A61K 31/4196*** *(2006.01)* ***A61K 31/437*** *(2006.01)*
***A61K 31/52*** *(2006.01)* ***A61P 35/00*** *(2006.01)*
***A61P 35/02*** *(2006.01)* ***A61P 35/04*** *(2006.01)*
***A61P 43/00*** *(2006.01)*

(86) International application number:
**PCT/JP2018/034092**

(87) International publication number:
**WO 2019/054465 (21.03.2019 Gazette 2019/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.09.2017 JP 2017178247**

(71) Applicant: **Taiho Pharmaceutical Co., Ltd.**
**Chiyoda-ku, Tokyo 101-8444 (JP)**

(72) Inventor: **MURAOKA, Hiromi**
**Tsukuba-shi**
**Ibaraki 300-2611 (JP)**

(74) Representative: **Blodig, Wolfgang**
**Wächtershäuser & Hartz**
**Patentanwaltspartnerschaft mbB**
**Weinstrasse 8**
**80333 München (DE)**

# (54) PROPHYLACTIC AND/OR THERAPEUTIC AGENT FOR DISEASES INVOLVING IDO EXPRESSION

(57) To provide a prophylactic and/or therapeutic agent for diseases involving IDO expression, and a pharmaceutical composition for treating IDO-positive tumors. A prophylactic and/or therapeutic agent for diseases involving IDO expression, comprising an HSP90-inhibiting compound as an active ingredient; and a pharmaceutical composition for treating IDO-positive tumors.

Figure 3

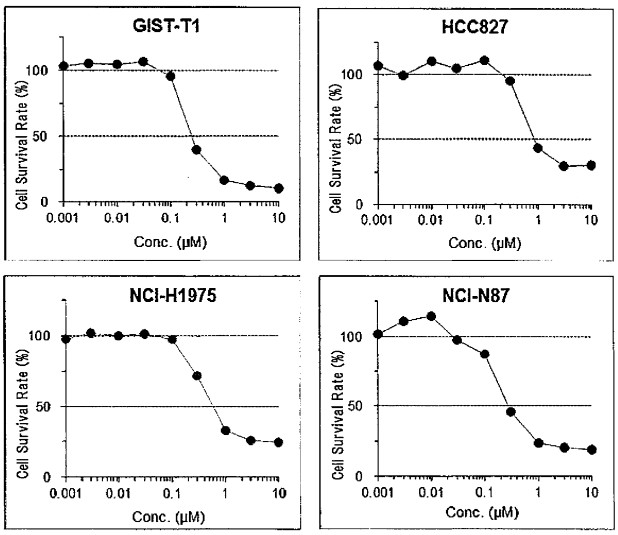




Printed by Jouve, 75001 PARIS (FR)

## Description

### Field of the Invention

The present invention relates to a prophylactic and/or therapeutic agent for diseases involving IDO expression, and a pharmaceutical composition for treating IDO-positive tumors.

### Background of the Invention

Indoleamine 2,3-dioxygenase (hereinafter, IDO) is a primary enzyme and rate-limiting enzyme in kynurenine degradation of tryptophane in a kynurenine pathway. IDO expressed by dendric cells affects proliferation and survival of T cells by locally depleting tryptophane to increase pro-apoptotic kynurenine. Thus, induction of IDO in dendric cells is an important immunosuppressive factor acting on immunological tolerance with regulatory T cells.

It is considered that in human cancer cells, activation of IDO is induced by IFNγ stimulus, and causes depletion of tryptophane to inhibit surrounding tumor immune cells, so that immune evasion occurs, resulting in maintenance of survival and proliferation of cancer cells. Further, according to the findings in clinical trial, high expression of IDO is associated with poor prognosis in chemotherapy and radiation therapy of cancers. In addition, clinical trials have been conducted on some IDO inhibitors, and indicated that IDO inhibitors may be useful against a wide range of cancers (Non Patent Literature 1). Further, effects on viral infections, neurodegenerative diseases, autoimmune diseases and the like have been indicated.

Thus, inhibition of IDO is considered to be useful as a prophylactic and/or therapeutic agent for diseases involving IDO expression.

On the other hand, heat shock protein (hereinafter, HSP) 90 is a molecular chaperone as abundant as approximately 1 to 2% of all intracellular soluble proteins. The molecular chaperon refers to a group of multifunctional proteins, which promotes the formation of the functional structures of other proteins or maintains these structures, promotes correct association, inhibits unnecessary aggregation, protects other proteins from degradation, and promotes secretion (Non Patent Literature 2). HSP90 is unnecessary for biosynthesis of the majority of polypeptides, unlike other chaperon proteins (Non Patent Literature 2).

HSP90 is deeply involved in cell proliferation or survival by maintaining the normal functions of client proteins (Non Patent Literatures 3 and 4). Further, HSP90 is required for the normal functions of mutated or chimeric factors (for example, BCR-ABL and NPM-ALK) which cause carcinogenesis or exacerbation of cancer. This indicates the importance of HSP90 particularly for processes such as carcinogenesis, cancer survival, growth, exacerbation, and metastasis (Non Patent Literature 3).

The inhibition of the chaperon functions of HSP90 by specific inhibitors such as geldanamycin causes the inactivation, destabilization, and degradation of the client proteins, resulting in induction of a halt in cell proliferation or apoptosis (Non Patent Literature 5). In terms of the physiological functions of HSP90, HSP90 inhibitors are characterized in that they can simultaneously inhibit multiple signaling pathways involved in cancer survival and growth. Thus, the HSP90 inhibitors can serve as pharmaceuticals having extensive and effective anticancer activity. Moreover, from the findings that cancer cell-derived HSP90 has higher activity and higher affinity for ATP or inhibitors than those of normal cell-derived HSP90, it has been expected that the HSP90 inhibitors would serve as pharmaceuticals having high cancer selectivity (Non Patent Literature 6).

The present applicant has reported HSP90 inhibitors having high inhibitory activity in Patent Literature 1.

However, it has not been heretofore known that an HSP90-inhibiting compound has IDO inhibitory action.

### Citation List

#### Patent Literature

Patent Literature 1: WO 2011/004610 A

#### Non Patent Literature

Non Patent Literature 1: Int. Immunopharmacol., 47: 70-77 (2017)
Non Patent Literature 2: Nat. Rev. Cancer, 5: 761-772 (2005)
Non Patent Literature 3: TRENDS Mol. Med., 10: 283-290 (2004)
Non Patent Literature 4: Clin. Cancer Res., 15: 9-14 (2009)

Non Patent Literature 5: Curr. Opin. Pharmacol., 8: 370-374 (2008)
Non Patent Literature 6: Drug Resist. Updat., 12: 17-27 (2009)

Summary of the Invention

Problems to be Solved by the Invention

An object of the invention is to provide a prophylactic and/or therapeutic agent for diseases involving IDO expression, and a pharmaceutical composition for treating IDO-positive tumors.

Means for Solving the Problem

The present inventor has conducted studies for discovering a prophylactic and/or therapeutic agent for diseases involving IDO expression, and resultantly found that an HSP90-inhibiting compound has excellent expression inhibitory activity against IDO.

Specifically, the present invention provides the following inventions [1] to [20].

[1] A prophylactic and/or therapeutic agent for diseases involving IDO expression comprising an HSP90-inhibiting compound as an active ingredient.

[2] The prophylactic and/or therapeutic agent for diseases involving IDO expression according to [1], wherein the HSP90-inhibiting compound is at least one member selected from the group consisting of an azabicyclo compound of the following Formula (I), tanespimycin, ganetespib, BIIB021 and a salt thereof:

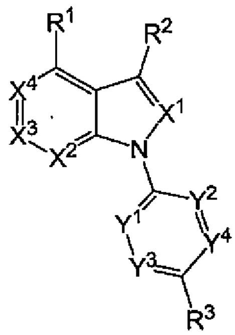

(I)

wherein, $X^1$ represents CH or N;
any one of $X^2$, $X^3$, and $X^4$ is N, and the others represent CH;
any one or two of $Y^1$, $Y^2$, $Y^3$, and $Y^4$ are C-$R^4$, and the others are the same or different and represent CH or N;
$R^1$ represents an optionally substituted mono- or bi-cyclic unsaturated heterocyclic group having 1 to 4 heteroatoms selected from the group consisting of N, S, and O;
$R^2$ represents a hydrogen atom, an optionally substituted alkyl group having 1 to 6 carbon atoms, or an optionally substituted alkenyl group having 2 to 6 carbon atoms;
$R^3$ represents a cyano group or -CO-$R^5$;
$R^4$(s) are the same or different and represent a hydrogen atom, a halogen atom, a cyano group, an optionally substituted alkyl group having 1 to 6 carbon atoms, an alkenyl group having 2 to 6 carbon atoms, an alkoxy group having 1 to 6 carbon atoms, an aromatic hydrocarbon group, -N($R^6$)($R^7$), -S-$R^8$, or -CO-$R^9$;
$R^5$ represents an amino group optionally having a hydroxyl group or an optionally substituted mono- or di-alkylamino group;
$R^6$ and $R^7$ are the same or different and represent a hydrogen atom, an optionally substituted alkyl group having 1 to 6 carbon atoms, a halogenoalkyl group having 1 to 6 carbon atoms, an optionally substituted cycloalkyl group having 3 to 7 carbon atoms, an optionally substituted aralkyl group, an optionally substituted aromatic hydrocarbon group, an optionally substituted saturated heterocyclic group, or an optionally substituted unsaturated heterocyclic group, or $R^6$ and $R^7$ optionally form a saturated heterocyclic group together with a nitrogen atom to which they are attached;
$R^8$ represents an optionally substituted cycloalkyl group having 3 to 7 carbon atoms or an optionally substituted aromatic hydrocarbon group; and
$R^9$ represents a hydrogen atom, a hydroxyl group, an amino group optionally having a hydroxyl group, or an optionally substituted mono- or di-alkylamino group.

[3] The prophylactic and/or therapeutic agent for diseases involving IDO expression according to [2], wherein the azabicyclo compound is a compound of Formula (I),
wherein, $X^1$ is CH or N;
$X^2$ is N and $X^3$ and $X^4$ are CH;
$Y^1$ and $Y^3$ are CH, any one or two of $Y^2$ and $Y^4$ are C-$R^4$, and the other is CH;
$R^1$ is any of an optionally substituted 1H-imidazol-1-yl group, an optionally substituted pyrazol-4-yl group, an optionally substituted thiophen-3-yl group, an optionally substituted furan-2-yl group, an optionally substituted pyridin-3-yl group, an optionally substituted pyridin-4-yl group, an optionally substituted indol-5-yl group, an optionally substituted 1H-pyrrolo[2,3-b]pyridin-5-yl group, an optionally substituted benzofuran-2-yl group, an optionally substituted quinolin-3-yl group, and an optionally substituted 5,6,7,8-tetrahydroquinolin-3-yl group;
$R^2$ is an alkyl group having 1 to 6 carbon atoms and optionally having a halogen atom or an alkenyl group having 2 to 6 carbon atoms;
$R^3$ is -CO-$R^5$;
$R^4$ is a halogen atom, an alkyl group having 1 to 6 carbon atoms and optionally having a mono- or di-(C1-C6 alkyl)amino group or a monocyclic 5- to 7-membered saturated heterocyclic group having one or two heteroatoms of any of N, S, and O, an alkoxy group having 1 to 6 carbon atoms, -N($R^6$)($R^7$), -S-$R^8$, or -CO-$R^9$;
$R^5$ is an amino group or mono- or di-(C1-C6 alkyl)amino group;
$R^6$ is a hydrogen atom or an optionally substituted alkyl group having 1 to 6 carbon atoms;
$R^7$ is a hydrogen atom, an optionally substituted alkyl group having 1 to 6 carbon atoms, an optionally substituted cycloalkyl group having 3 to 7 carbon atoms, an optionally substituted aralkyl group having 7 to 12 carbon atoms, an optionally substituted aromatic hydrocarbon group having 6 to 14 carbon atoms, an optionally substituted mono- or bi-cyclic saturated heterocyclic group having 1 to 4 heteroatoms selected from the group consisting of N, S, and O, or an optionally substituted mono- or bi-cyclic unsaturated heterocyclic group having 1 to 4 heteroatoms selected from the group consisting of N, S, and O, or $R^6$ and $R^7$ form a 5- to 7-membered saturated heterocyclic group together with a nitrogen atom to which they are attached;
$R^8$ is an optionally substituted cycloalkyl group having 3 to 7 carbon atoms or an optionally substituted aromatic hydrocarbon group having 6 to 14 carbon atoms; and
$R^9$ is a hydrogen atom, a hydroxyl group, an amino group, or a mono- or di-(C1-C6 alkyl)amino group.
[4] The prophylactic and/or therapeutic agent for diseases involving IDO expression according to [2] or [3], wherein the azabicyclo compound is 3-ethyl-4-{3-isopropyl-4-(4-(1-methyl-1H-pyrazol-4-yl)-1H-imidazol-1-yl)-1H-pyrazolo[3,4-b]pyridin-1-yl}benzamide.
[5] A pharmaceutical composition for treating IDO-positive tumors, comprising an HSP90-inhibiting compound as an active ingredient.
[6] An HSP90-inhibiting compound for use in preventing and/or treating diseases involving IDO expression.
[7] The HSP90-inhibiting compound according to [6], wherein the HSP90-inhibiting compound is at least one member selected from the group consisting of an azabicyclo compound of the following Formula (I), tanespimycin, ganetespib, BIIB021 and a salt thereof:

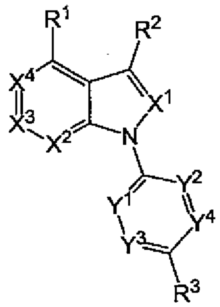

(I)

wherein, $X^1$ represents CH or N;
any one of $X^2$, $X^3$, and $X^4$ is N, and the others represent CH;
any one or two of $Y^1$, $Y^2$, $Y^3$, and $Y^4$ are C-$R^4$, and the others are the same or different and represent CH or N;
$R^1$ represents an optionally substituted mono- or bi-cyclic unsaturated heterocyclic group having 1 to 4 heteroatoms selected from the group consisting of N, S, and O;
$R^2$ represents a hydrogen atom, an optionally substituted alkyl group having 1 to 6 carbon atoms, or an optionally substituted alkenyl group having 2 to 6 carbon atoms;
$R^3$ represents a cyano group or -CO-$R^5$;
$R^4$(s) are the same or different and represent a hydrogen atom, a halogen atom, a cyano group, an optionally

substituted alkyl group having 1 to 6 carbon atoms, an alkenyl group having 2 to 6 carbon atoms, an alkoxy group having 1 to 6 carbon atoms, an aromatic hydrocarbon group, $-N(R^6)(R^7)$, $-S-R^8$, or $-CO-R^9$;

$R^5$ represents an amino group optionally having a hydroxyl group or an optionally substituted mono- or di-alkylamino group;

$R^6$ and $R^7$ are the same or different and represent a hydrogen atom, an optionally substituted alkyl group having 1 to 6 carbon atoms, a halogenoalkyl group having 1 to 6 carbon atoms, an optionally substituted cycloalkyl group having 3 to 7 carbon atoms, an optionally substituted aralkyl group, an optionally substituted aromatic hydrocarbon group, an optionally substituted saturated heterocyclic group, or an optionally substituted unsaturated heterocyclic group, or $R^6$ and $R^7$ optionally form a saturated heterocyclic group together with a nitrogen atom to which they are attached;

$R^8$ represents an optionally substituted cycloalkyl group having 3 to 7 carbon atoms or an optionally substituted aromatic hydrocarbon group; and

$R^9$ represents a hydrogen atom, a hydroxyl group, an amino group optionally having a hydroxyl group, or an optionally substituted mono- or di-alkylamino group.

[8] The HSP90-inhibiting compound according to [7], wherein the azabicyclo compound is a compound of Formula (I), wherein, $X^1$ is CH or N;

$X^2$ is N and $X^3$ and $X^4$ are CH;

$Y^1$ and $Y^3$ are CH, any one or two of $Y^2$ and $Y^4$ are $C-R^4$, and the other is CH;

$R^1$ is any of an optionally substituted 1H-imidazol-1-yl group, an optionally substituted pyrazol-4-yl group, an optionally substituted thiophen-3-yl group, an optionally substituted furan-2-yl group, an optionally substituted pyridin-3-yl group, an optionally substituted pyridin-4-yl group, an optionally substituted indol-5-yl group, an optionally substituted 1H-pyrrolo[2,3-b]pyridin-5-yl group, an optionally substituted benzofuran-2-yl group, an optionally substituted quinolin-3-yl group, and an optionally substituted 5,6,7,8-tetrahydroquinolin-3-yl group;

$R^2$ is an alkyl group having 1 to 6 carbon atoms and optionally having a halogen atom or an alkenyl group having 2 to 6 carbon atoms;

$R^3$ is $-CO-R^5$;

$R^4$ is a halogen atom, an alkyl group having 1 to 6 carbon atoms and optionally having a mono- or di-(C1-C6 alkyl)amino group or a monocyclic 5- to 7-membered saturated heterocyclic group having one or two heteroatoms of any one of N, S, and O, an alkoxy group having 1 to 6 carbon atoms, $-N(R^6)(R^7)$, $-S-R^8$, or $-CO-R^9$;

$R^5$ is an amino group or mono- or di-(C1-C6 alkyl)amino group;

$R^6$ is a hydrogen atom or an optionally substituted alkyl group having 1 to 6 carbon atoms;

$R^7$ is a hydrogen atom, an optionally substituted alkyl group having 1 to 6 carbon atoms, an optionally substituted cycloalkyl group having 3 to 7 carbon atoms, an optionally substituted aralkyl group having 7 to 12 carbon atoms, an optionally substituted aromatic hydrocarbon group having 6 to 14 carbon atoms, an optionally substituted mono- or bi-cyclic saturated heterocyclic group having 1 to 4 heteroatoms selected from the group consisting of N, S, and O, or an optionally substituted mono- or bi-cyclic unsaturated heterocyclic group having 1 to 4 heteroatoms selected from the group consisting of N, S, and O, or $R^6$ and $R^7$ form a 5- to 7-membered saturated heterocyclic group together with a nitrogen atom to which they are attached;

$R^8$ is an optionally substituted cycloalkyl group having 3 to 7 carbon atoms or an optionally substituted aromatic hydrocarbon group having 6 to 14 carbon atoms; and

$R^9$ is a hydrogen atom, a hydroxyl group, an amino group, or a mono- or di-(C1-C6 alkyl)amino group.

[9] The HSP90-inhibiting compound according to [7] or [8], wherein the azabicyclo compound is 3-ethyl-4-{3-isopropyl-4-(4-(1-methyl-1H-pyrazol-4-yl)-1H-imidazol-1-yl)-1H-pyrazolo[3,4-b]pyridin-1-yl}benzamide.

[10] An HSP90-inhibiting compound which is used as a pharmaceutical composition for treating IDO-positive tumors.

[11] Use of an HSP90-inhibiting compound for producing a prophylactic and/or therapeutic agent for diseases involving IDO expression.

[12] The use according to [11], wherein the HSP90-inhibiting compound is at least one member selected from the group consisting of an azabicyclo compound of the following Formula (I), tanespimycin, ganetespib, BIIB021 and a salt thereof:

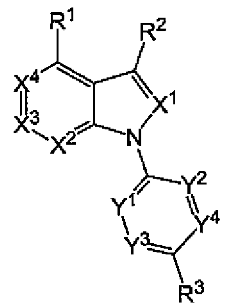

(I)

wherein, $X^1$ represents CH or N;
any one of $X^2$, $X^3$, and $X^4$ is N, and the others represent CH;
any one or two of $Y^1$, $Y^2$, $Y^3$, and $Y^4$ are C-$R^4$, and the others are the same or different and represent CH or N;
$R^1$ represents an optionally substituted mono- or bi-cyclic unsaturated heterocyclic group having 1 to 4 heteroatoms selected from the group consisting of N, S, and O;
$R^2$ represents a hydrogen atom, an optionally substituted alkyl group having 1 to 6 carbon atoms, or an optionally substituted alkenyl group having 2 to 6 carbon atoms;
$R^3$ represents a cyano group or -CO-$R^5$;
$R^4$(s) are the same or different and represent a hydrogen atom, a halogen atom, a cyano group, an optionally substituted alkyl group having 1 to 6 carbon atoms, an alkenyl group having 2 to 6 carbon atoms, an alkoxy group having 1 to 6 carbon atoms, an aromatic hydrocarbon group, -N($R^6$)($R^7$), -S-$R^8$, or -CO-$R^9$;
$R^5$ represents an amino group optionally having a hydroxyl group or an optionally substituted mono- or di-alkylamino group;
$R^6$ and $R^7$ are the same or different and represent a hydrogen atom, an optionally substituted alkyl group having 1 to 6 carbon atoms, a halogenoalkyl group having 1 to 6 carbon atoms, an optionally substituted cycloalkyl group having 3 to 7 carbon atoms, an optionally substituted aralkyl group, an optionally substituted aromatic hydrocarbon group, an optionally substituted saturated heterocyclic group, or an optionally substituted unsaturated heterocyclic group, or $R^6$ and $R^7$ optionally form a saturated heterocyclic group together with a nitrogen atom to which they are attached;
$R^8$ represents an optionally substituted cycloalkyl group having 3 to 7 carbon atoms or an optionally substituted aromatic hydrocarbon group; and
$R^9$ represents a hydrogen atom, a hydroxyl group, an amino group optionally having a hydroxyl group, or an optionally substituted mono- or di-alkylamino group.

[13] The use according to [12], wherein the azabicyclo compound is a compound of Formula (I),
wherein, $X^1$ is CH or N;
$X^2$ is N and $X^3$ and $X^4$ are CH;
$Y^1$ and $Y^3$ are CH, any one or two of $Y^2$ and $Y^4$ are C-$R^4$, and the other is CH;
$R^1$ is any of an optionally substituted 1H-imidazol-1-yl group, an optionally substituted pyrazol-4-yl group, an optionally substituted thiophen-3-yl group, an optionally substituted furan-2-yl group, an optionally substituted pyridin-3-yl group, an optionally substituted pyridin-4-yl group, an optionally substituted indol-5-yl group, an optionally substituted 1H-pyrrolo[2,3-b]pyridin-5-yl group, an optionally substituted benzofuran-2-yl group, an optionally substituted quinolin-3-yl group, and an optionally substituted 5,6,7,8-tetrahydroquinolin-3-yl group;
$R^2$ is an alkyl group having 1 to 6 carbon atoms and optionally having a halogen atom or an alkenyl group having 2 to 6 carbon atoms;
$R^3$ is -CO-$R^5$;
$R^4$ is a halogen atom, an alkyl group having 1 to 6 carbon atoms and optionally having a mono- or di-(C1-C6 alkyl)amino group or a monocyclic 5- to 7-membered saturated heterocyclic group having one or two heteroatoms of any of N, S, and O, an alkoxy group having 1 to 6 carbon atoms, -N($R^6$)($R^7$), -S-$R^8$, or -CO-$R^9$;
$R^5$ is an amino group or mono- or di-(C1-C6 alkyl)amino group;
$R^6$ is a hydrogen atom or an optionally substituted alkyl group having 1 to 6 carbon atoms;
$R^7$ is a hydrogen atom, an optionally substituted alkyl group having 1 to 6 carbon atoms, an optionally substituted cycloalkyl group having 3 to 7 carbon atoms, an optionally substituted aralkyl group having 7 to 12 carbon atoms, an optionally substituted aromatic hydrocarbon group having 6 to 14 carbon atoms, an optionally substituted mono- or bi-cyclic saturated heterocyclic group having 1 to 4 heteroatoms selected from the group consisting of N, S, and O, or an optionally substituted mono- or bi-cyclic unsaturated heterocyclic group having 1 to 4 heteroatoms selected from the group consisting of N, S, and O, or $R^6$ and $R^7$ form a 5- to 7-membered saturated heterocyclic group

together with a nitrogen atom to which they are attached;
$R^8$ is an optionally substituted cycloalkyl group having 3 to 7 carbon atoms or an optionally substituted aromatic hydrocarbon group having 6 to 14 carbon atoms; and
$R^9$ is a hydrogen atom, a hydroxyl group, an amino group, or a mono- or di-(C1-C6 alkyl)amino group.
[14] The use according to [12] or [13], wherein the azabicyclo compound is 3-ethyl-4-{3-isopropyl-4-(4-(1-methyl-1H-pyrazol-4-yl)-1H-imidazol-1-yl)-1H-pyrazolo[3,4-b]pyridin-1-yl}benzamide.
[15] Use of an HSP90-inhibiting compound for producing a pharmaceutical composition for treating IDO-positive tumors.
[16] A method for preventing and/or treating diseases involving IDO expression, the method comprising administering to a subject in need thereof an effective amount of an HSP90-inhibiting compound.
[17] The method according to [16], wherein the HSP90-inhibiting compound is at least one member selected from the group consisting of an azabicyclo compound of the following Formula (I), tanespimycin, ganetespib, BIIB021 and a salt thereof:

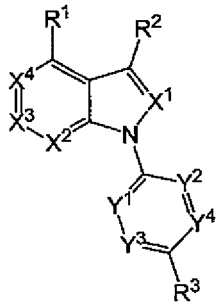

(I)

wherein, $X^1$ represents CH or N;
any one of $X^2$, $X^3$, and $X^4$ is N, and the others represent CH;
any one or two of $Y^1$, $Y^2$, $Y^3$, and $Y^4$ are C-$R^4$, and the others are the same or different and represent CH or N;
$R^1$ represents an optionally substituted mono- or bi-cyclic unsaturated heterocyclic group having 1 to 4 heteroatoms selected from the group consisting of N, S, and O;
$R^2$ represents a hydrogen atom, an optionally substituted alkyl group having 1 to 6 carbon atoms, or an optionally substituted alkenyl group having 2 to 6 carbon atoms;
$R^3$ represents a cyano group or -CO-$R^5$;
$R^4$(s) are the same or different and represent a hydrogen atom, a halogen atom, a cyano group, an optionally substituted alkyl group having 1 to 6 carbon atoms, an alkenyl group having 2 to 6 carbon atoms, an alkoxy group having 1 to 6 carbon atoms, an aromatic hydrocarbon group, -N($R^6$)($R^7$), -S-$R^8$, or -CO-$R^9$;
$R^5$ represents an amino group optionally having a hydroxyl group or an optionally substituted mono- or di-alkylamino group;
$R^6$ and $R^7$ are the same or different and represent a hydrogen atom, an optionally substituted alkyl group having 1 to 6 carbon atoms, a halogenoalkyl group having 1 to 6 carbon atoms, an optionally substituted cycloalkyl group having 3 to 7 carbon atoms, an optionally substituted aralkyl group, an optionally substituted aromatic hydrocarbon group, an optionally substituted saturated heterocyclic group, or an optionally substituted unsaturated heterocyclic group, or $R^6$ and $R^7$ optionally form a saturated heterocyclic group together with a nitrogen atom to which they are attached;
$R^8$ represents an optionally substituted cycloalkyl group having 3 to 7 carbon atoms or an optionally substituted aromatic hydrocarbon group; and
$R^9$ represents a hydrogen atom, a hydroxyl group, an amino group optionally having a hydroxyl group, or an optionally substituted mono- or di-alkylamino group.

[18] The method according to [17], wherein the azabicyclo compound is a compound of Formula (I),
wherein, $X^1$ is CH or N;
$X^2$ is N and $X^3$ and $X^4$ are CH;
$Y^1$ and $Y^3$ are CH, any one or two of $Y^2$ and $Y^4$ are C-$R^4$, and the other is CH;
$R^1$ is any of an optionally substituted 1H-imidazol-1-yl group, an optionally substituted pyrazol-4-yl group, an optionally substituted thiophen-3-yl group, an optionally substituted furan-2-yl group, an optionally substituted pyridin-3-yl group, an optionally substituted pyridin-4-yl group, an optionally substituted indol-5-yl group, an optionally substituted 1H-pyrrolo[2,3-b]pyridin-5-yl group, an optionally substituted benzofuran-2-yl group, an optionally substituted quinolin-3-yl group, and an optionally substituted 5,6,7,8-tetrahydroquinolin-3-yl group;

$R^2$ is an alkyl group having 1 to 6 carbon atoms and optionally having a halogen atom or an alkenyl group having 2 to 6 carbon atoms;
$R^3$ is -CO-$R^5$;
$R^4$ is a halogen atom, an alkyl group having 1 to 6 carbon atoms and optionally having a mono- or di-(C1-C6 alkyl)amino group or a monocyclic 5- to 7-membered saturated heterocyclic group having one or two heteroatoms of any of N, S, and O, an alkoxy group having 1 to 6 carbon atoms, -N($R^6$)($R^7$), -S-$R^8$, or -CO-$R^9$;
$R^5$ is an amino group or mono- or di-(C1-C6 alkyl)amino group;
$R^6$ is a hydrogen atom or an optionally substituted alkyl group having 1 to 6 carbon atoms;
$R^7$ is a hydrogen atom, an optionally substituted alkyl group having 1 to 6 carbon atoms, an optionally substituted cycloalkyl group having 3 to 7 carbon atoms, an optionally substituted aralkyl group having 7 to 12 carbon atoms, an optionally substituted aromatic hydrocarbon group having 6 to 14 carbon atoms, an optionally substituted mono- or bi-cyclic saturated heterocyclic group having 1 to 4 heteroatoms selected from the group consisting of N, S, and O, or an optionally substituted mono- or bi-cyclic unsaturated heterocyclic group having 1 to 4 heteroatoms selected from the group consisting of N, S, and O, or $R^6$ and $R^7$ form a 5- to 7-membered saturated heterocyclic group together with a nitrogen atom to which they are attached;
$R^8$ is an optionally substituted cycloalkyl group having 3 to 7 carbon atoms or an optionally substituted aromatic hydrocarbon group having 6 to 14 carbon atoms; and
$R^9$ is a hydrogen atom, a hydroxyl group, an amino group, or a mono- or di-(C1-C6 alkyl)amino group.
[19] The method according to [17] or [18], wherein the azabicyclo compound is 3-ethyl-4-{3-isopropyl-4-(4-(1-methyl-1H-pyrazol-4-yl)-1H-imidazol-1-yl)-1H-pyrazolo[3,4-b]pyridin-1-yl}benzamide.
[20] A method for preventing and/or treating IDO-positive tumors, the method comprising administering to a subject in need thereof an effective amount of an HSP90-inhibiting compound.

The invention also relates to the following aspects.

- An HSP90-inhibiting compound for preventing and/or treating diseases involving IDO expression.
- Use of an HSP90-inhibiting compound for preventing and/or treating diseases involving IDO expression.
- Use of an HSP90-inhibiting compound for producing a prophylactic and/or therapeutic agent for diseases involving IDO expression.
- An HSP90-inhibiting compound for treating IDO-positive tumors.
- Use of an HSP90-inhibiting compound for treating IDO-positive tumors.
- A method for preventing and/or treating diseases involving IDO expression, the method comprising the step of administering an effective amount of an HSP90-inhibiting compound to a patient in need of prevention and/or treatment of a disease involving IDO expression.
- Use of an HSP90-inhibiting compound for producing a pharmaceutical composition for treating IDO-positive tumors.
- A method for preventing and/or treating IDO-positive tumors, the method comprising the step of administering a prophylactically and/or therapeutically effective amount of an HSP90-inhibiting compound.
- An IDO inhibitor having an HSP90-inhibiting compound as an active ingredient.
- A method for inhibiting IDO, the method comprising the step of administering to a subject in need thereof an effective amount of an HSP90-inhibiting compound.
- An HSP90-inhibiting compound for use in inhibition of IDO.

## Advantageous Effects of the Invention

The prophylactic and/or therapeutic agent for diseases involving IDO expression according to the present invention serves to provide novel therapy for treating diseases which can be improved by inhibitory action on IDO expression, and IDO-positive tumors.

## Brief Description of Drawings

[Figure 1] Figure 1 shows inhibitory effects on IDO expression by use of HSP90-inhibiting compounds.
[Figure 2] Figure 2 shows IDO expression in each cell line to which IFNγ was added.
[Figure 3] Figure 3 shows suppressive effects of Compound 1 on cell proliferation in IDO-positive cells.
[Figure 4] Figure 4 shows HSP90 expression in each cell line to which IFNγ was added.

Detailed Description of the Invention

The present invention relates to a prophylactic and/or therapeutic agent for diseases involving IDO expression, which contains an HSP90-inhibiting compound as an active ingredient; and a pharmaceutical composition for treating IDO-positive tumors.

The "indoleamine 2,3-dioxygenase (IDO)" according to the present invention is a protein also referred to as IDO1, and includes human or non-human mammal IDO proteins. The IDO protein is preferably human IDO protein.

In the present invention, the "IDO protein" includes isoforms which are splice variants thereof, and examples thereof derived from a human include protein consisting of an amino acid sequence set forth as GenPept accession No: NP_002155. Further, in the present invention, the "IDO gene" includes a gene consisting of a nucleotide sequence encoding the amino acid sequence, and examples thereof derived from a human include a gene consisting of the nucleotide sequences set forth as GenBank accession No. NM_002164.

In the present invention, the "HSP90-inhibiting compound" is a compound which inhibits HSP90 activity. As described above, HSP90 is a heat shock protein functioning as a molecular chaperone and having a molecular weight of 90 kDa. The "HSP90 activity" can be examined by, for example, labeling a compound (geldanamycin or the like) to be attached to HSP90 at the ATP binding site and measuring an inhibitory effect on binding of the labeled compound in an appropriate HSP90 binding inhibition assay. The HSP90 activity can be evaluated according to the method described in Test Example 1 in WO 2011/004610, for example.

Examples of the HSP90-inhibiting compounds include azabicyclo compounds of the following Formula (I), geldanamycin, tanespimycin, alvespimycin, luminespib, ganetespib, onalespib, CUDC-305, DS-2248, SNX-2112, SNX-5422, KW-2478, AB-010, XL888, MPC-3100, PU-H71, BIIB021, BIIB028 and salts thereof.

The HSP90-exhibiting compound is preferably an azabicyclo compound of the following Formula (I), tanespimycin, ganetespib, BIIB021 or a salt thereof, more preferably an azabicyclo compound of the following Formula (I) or a salt thereof.

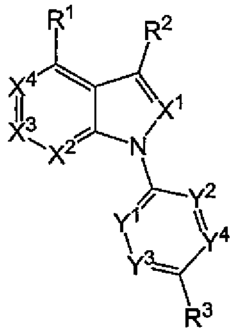

(I)

wherein, $X^1$ represents CH or N;

any one of $X^2$, $X^3$, and $X^4$ is N, and the others represent CH;

any one or two of $Y^1$, $Y^2$, $Y^3$, and $Y^4$ are C-$R^4$, and the others are the same or different and represent CH or N;

$R^1$ represents an optionally substituted mono- or bi-cyclic unsaturated heterocyclic group having 1 to 4 heteroatoms selected from the group consisting of N, S, and O;

$R^2$ represents a hydrogen atom, an optionally substituted alkyl group having 1 to 6 carbon atoms, or an optionally substituted alkenyl group having 2 to 6 carbon atoms;

$R^3$ represents a cyano group or -CO-$R^5$;

$R^4$(s) are the same or different and represent a hydrogen atom, a halogen atom, a cyano group, an optionally substituted alkyl group having 1 to 6 carbon atoms, an alkenyl group having 2 to 6 carbon atoms, an alkoxy group having 1 to 6 carbon atoms, an aromatic hydrocarbon group, -N($R^6$)($R^7$), -S-$R^8$, or -CO-$R^9$;

$R^5$ represents an amino group optionally having a hydroxyl group or an optionally substituted mono- or di-alkylamino group;

$R^6$ and $R^7$ are the same or different and represent a hydrogen atom, an optionally substituted alkyl group having 1 to 6 carbon atoms, a halogenoalkyl group having 1 to 6 carbon atoms, an optionally substituted cycloalkyl group having 3 to 7 carbon atoms, an optionally substituted aralkyl group, an optionally substituted aromatic hydrocarbon group, an optionally substituted saturated heterocyclic group, or an optionally substituted unsaturated heterocyclic group, or $R^6$ and $R^7$ optionally form a saturated heterocyclic group together with a nitrogen atom to which they are attached;

$R^8$ represents an optionally substituted cycloalkyl group having 3 to 7 carbon atoms or an optionally substituted aromatic hydrocarbon group; and

$R^9$ represents a hydrogen atom, a hydroxyl group, an amino group optionally having a hydroxyl group, or an optionally substituted mono- or di-alkylamino group.

In the present specification, examples of the "substituent(s)" include a halogen atom, a hydroxyl group, a cyano group, a nitro group, an alkyl group, a halogenoalkyl group, a cycloalkyl group, a cycloalkyl-alkyl group, an aralkyl group, a hydroxyalkyl group, an alkenyl group, an alkynyl group, an alkoxy group, a halogenoalkoxy group, an alkoxy-alkyl group, a cycloalkoxy group, a cycloalkyl-alkoxy group, an aralkyloxy group, an aralkyloxy-alkyl group, an alkylthio group, a cycloalkyl-alkylthio group, an amino group, a mono- or dialkylamino group, a cycloalkyl-alkylamino group, an acyl group, an acyloxy group, an oxo group, a carboxyl group, an alkoxycarbonyl group, an aralkyloxycarbonyl group, a carbamoyl group, a saturated or unsaturated heterocyclic group, an aromatic hydrocarbon group, and a saturated heterocyclic oxy group. When the above substituent is present, the number of the substituents is typically 1 to 3.

Examples of the halogen atom included in the substituent(s) include a chlorine atom, a bromine atom, a fluorine atom, and an iodine atom.

The alkyl group or the halogenoalkyl group included in the substituents preferably refers to a linear or branched alkyl group having 1 to 6 carbon atoms or a group in which one to all hydrogen atom(s) in such an alkyl group are substituted by the halogen atom described above. Examples thereof include alkyl groups such as a methyl group, an ethyl group, an n-propyl group, an isopropyl group, an n-butyl group, an isobutyl group, a sec-butyl group, a tert-butyl group, a pentyl group, and a hexyl group and halogenoalkyl groups such as a trifluoromethyl group.

The cycloalkyl group included in the substituents is preferably a cycloalkyl group having 3 to 7 carbon atoms, and examples thereof include a cyclopropyl group, a cyclobutyl group, a cyclopentyl group, a cyclohexyl group, and a cycloheptyl group.

The cycloalkyl-alkyl group included in the substituents is preferably an alkyl group having 1 to 6 carbon atoms which is substituted by cycloalkyl having 3 to 7 carbon atoms, and examples thereof include a cyclopropylmethyl group, a cyclopropylethyl group, a cyclobutylmethyl group, a cyclopentylmethyl group, and a cyclohexylmethyl group.

The aralkyl group included in the substituents preferably refers to a linear or branched alkyl group having 1 to 6 carbon atoms which is substituted by an aromatic hydrocarbon group having 6 to 14 carbon atoms, and examples thereof include a benzyl group, a phenylethyl group, a phenylpropyl group, a naphthylmethyl group, and a naphthylethyl group.

The hydroxyalkyl group included in the substituents preferably refers to the linear or branched alkyl group having 1 to 6 carbon atoms described above which has a hydroxy group, and examples thereof include a hydroxymethyl group and a hydroxyethyl group.

The alkenyl group included in the substituents preferably refers to an alkenyl group having 2 to 6 carbon atoms which contains a carbon-carbon double bond, and examples thereof include a vinyl group, an allyl group, a methylvinyl group, a propenyl group, a butenyl group, a pentenyl group, and a hexenyl group.

The alkynyl group included in the substituents preferably refers to an alkynyl group having 2 to 6 carbon atoms which contains a carbon-carbon triple bond, and examples thereof include an ethynyl group and a propargyl group.

The alkoxy group or the halogenoalkoxy group included in the substituents preferably refers to a linear or branched alkoxy group having 1 to 6 carbon atoms, or a group in which such an alkoxy group is substituted by the halogen atom described above, and examples thereof include a methoxy group, an ethoxy group, an n-propoxy group, an isopropoxy group, a 1-methylpropoxy group, an n-butoxy group, an isobutoxy group, a tert-butoxy group, a 2-methylbutoxy group, a neopentyloxy group, a pentan-2-yloxy group, a fluoromethoxy group, a difluoromethoxy group, a trifluoromethoxy group, a 1,1-difluoroethoxy group, a 2,2-difluoroethoxy group, a 2,2,2-trifluoroethoxy group, a 1,1,2,2-tetrafluoroethoxy group, a perfluoroethoxy group, a 3-fluoro-2-(fluoromethyl)-propoxy group, a 1,3-difluoropropan-2-yloxy group, and a 2,2,3,3,3-pentafluoro-1-propoxy group.

The cycloalkoxy group included in the substituents is preferably a cycloalkoxy group having 3 to 7 carbon atoms, and examples thereof include a cyclopropoxy group, a cyclobutoxy group, a cyclopentyloxy group, a cyclohexyloxy group, and a cycloheptyloxy group.

The alkoxy-alkyl group included in the substituents preferably refers to the alkyl group having 1 to 6 carbon atoms described above which is substituted by the linear or branched alkoxy group having 1 to 6 carbon atoms described above, and examples thereof include a methoxymethyl group and an ethoxymethyl group.

The cycloalkyl-alkoxy group included in the substituents is preferably an alkoxy group having 1 to 6 carbon atoms which is substituted by cycloalkyl having 3 to 7 carbon atoms, and examples thereof include a cyclopropylmethoxy group, a cyclopropylethoxy group, a cyclobutylmethoxy group, a cyclopentylmethoxy group, and a cyclohexylmethoxy group.

The aralkyloxy group included in the substituents preferably refers to an oxy group which has the aralkyl group described above, and examples thereof include a benzyloxy group, a phenethyloxy group, a phenylpropyloxy group, a naphthylmethyloxy group, and a naphthylethyloxy group.

The aralkyloxy-alkyl group included in the substituents preferably refers to the linear or branched alkyl group

having 1 to 6 carbon atoms described above which has the aralkyloxy group described above, and examples thereof include a benzyloxymethyl group and a benzyloxyethyl group.

The alkylthio group included in the substituents is preferably a (C1-C6) alkylthio group which refers to a linear or branched alkylthio group having 1 to 6 carbon atoms, and examples thereof include a methylthio group, an ethylthio group, an n-propylthio group, an isopropylthio group, an n-butylthio group, an isobutylthio group, a sec-butylthio group, a tert-butylthio group, a pentylthio group, and a hexylthio group.

The cycloalkyl-alkylthio group included in the substituents is preferably an alkylthio group having 1 to 6 carbon atoms which is substituted by cycloalkyl having 3 to 7 carbon atoms, and examples thereof include a cyclopropylmethylthio group, a cyclopropylethylthio group, a cyclobutylmethylthio group, a cyclopentylmethylthio group, and a cyclohexylmethylthio group.

The mono- or dialkylamino group included in the substituents is a mono- or di-(C1-C6 alkyl)amino group which refers to an amino group which is monosubstituted or disubstituted by the linear or branched alkyl group having 1 to 6 carbon atoms described above, and examples thereof include a methylamino group, a dimethylamino group, an ethylamino group, a diethylamino group, and a methylethylamino group.

The cycloalkyl-alkylamino group included in the substituents refers to an alkylamino group which is substituted by the cycloalkyl group described above, and examples thereof include a cyclopropylmethylamino group, a cyclobutylmethylamino group, and a cyclopentylmethylamino group.

Examples of the acyl group included in the substituents include: linear or branched acyl groups having 1 to 6 carbon atoms such as a formyl group, an acetyl group, a propionyl group, an n-butyryl group, an isobutyryl group, a valeryl group, an isovaleryl group, and a pivaloyl group; and a benzoyl group.

Examples of the acyloxy group included in the substituents include: linear or branched acyloxy groups having 1 to 6 carbon atoms such as a formyloxy group, an acetoxy group, a propionyloxy group, an n-butyryloxy group, an isobutyryloxy group, a valeryloxy group, an isovaleryloxy group, and a pivaloyloxy group; a benzoyloxy group; and amino acid-derived acyloxy groups such as a glycyloxy group, an alanyloxy group, and a leucyloxy group.

The alkoxycarbonyl group included in the substituents refers to a carbonyl group which is substituted by the alkoxy group described above, and examples thereof include a methoxycarbonyl group, an ethoxycarbonyl group, an n-propoxycarbonyl group, an isopropoxycarbonyl group, a 1-methylpropoxycarbonyl group, an n-butoxycarbonyl group, an isobutoxycarbonyl group, a tert-butoxycarbonyl group, a 2-methyl-butoxycarbonyl group, a neopentyloxycarbonyl group, and a pentan-2-yloxycarbonyl group.

The aralkyloxycarbonyl group included in the substituents preferably refers to a carbonyl group which is substituted by the aralkyloxy group described above, and examples thereof include a benzyloxycarbonyl group, a phenethyloxycarbonyl group, a phenylpropyloxycarbonyl group, a naphthylmethyloxycarbonyl group, and a naphthylethyloxycarbonyl group.

Examples of the carbamoyl group included in the substituents include a -CONH2 group, a (mono- or dialkyl)carbamoyl group, a (mono- or diaryl)carbamoyl group, an (N-alkyl-N-aryl)carbamoyl group, a pyrrolidinocarbamoyl group, a piperidinocarbamoyl group, a piperazinocarbamoyl group, and a morpholinocarbamoyl group.

The saturated or unsaturated heterocyclic group included in the substituents refers to a mono- or bi-cyclic saturated or 5- to 10-membered unsaturated heterocyclic group preferably having 1 to 4 heteroatoms of any one of N, S and O, and examples thereof include a pyrrolidinyl group, a piperidinyl group, a piperazinyl group, a hexamethyleneimino group, a morpholino group, a thiomorpholino group, a homopiperazinyl group, a tetrahydrofuranyl group, a tetrahydropyranyl group, an imidazolyl group, a thienyl group, a furyl group, a pyrrolyl group, an oxazolyl group, an isoxazolyl group, a thiazolyl group, an isothiazolyl group, a pyrazolyl group, a triazolyl group, a tetrazolyl group, a pyridyl group, a pyrazyl group, a pyrimidinyl group, a pyridazinyl group, an indolyl group, an isoindolyl group, an indazolyl group, a methylenedioxyphenyl group, an ethylenedioxyphenyl group, a benzofuranyl group, a dihydrobenzofuranyl group, a benzoimidazolyl group, a benzooxazolyl group, a benzothiazolyl group, a purinyl group, a quinolyl group, an isoquinolyl group, a quinazolinyl group, and a quinoxalyl group.

The aromatic hydrocarbon group included in the substituents preferably refers to an aromatic hydrocarbon group having 6 to 14 carbon atoms, and examples thereof include a phenyl group and a naphthyl group.

The saturated heterocyclic oxy group included in the substituents refers to a monocyclic 5- to 7-membered saturated heterocyclic group having one or two heteroatoms of any of N, S and O, for example, an oxy group which has a pyrrolidinyl group, a piperidinyl group, a piperazinyl group, a hexamethyleneimino group, a morpholino group, a thiomorpholino group, or a homopiperazinyl group. Examples thereof include a tetrahydrofuranyloxy group and a tetrahydropyranyloxy group.

In Formula (I), $X^1$ represents CH or N. Moreover, in Formula (I), any one of $X^2$, $X^3$, and $X^4$ represents N, and the others represent CH. Based on the definitions of $X^1$ to $X^4$, examples of the azabicyclo skeleton in Formula (I) include the following structures:

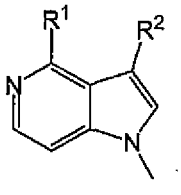 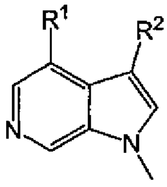 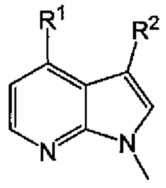

(A−1) (A−2) (A−3)

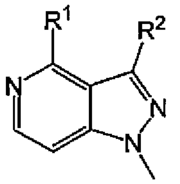 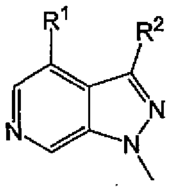 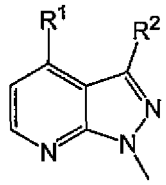

(A−4) (A−5) (A−6)

wherein, $R^1$ and $R^2$ are as defined above.

Among these skeletons, (A-3) and (A-6) are particularly preferable.

In Formula (I), the "mono- or bi-cyclic unsaturated heterocyclic group having 1 to 4 heteroatoms selected from the group consisting of N, S, and O" in the "optionally substituted mono- or bi-cyclic unsaturated heterocyclic group having 1 to 4 heteroatoms selected from the group consisting of N, S, and O" represented by $R^1$ is preferably a mono- or bi-cyclic 5- to 10-membered unsaturated heterocyclic group having 1 to 3 heteroatoms selected from the group consisting of N, S, and O, more preferably a monocyclic 5- to 6-membered unsaturated heterocyclic group having 1 to 3 heteroatoms selected from the group consisting of N, S, and O, or a bicyclic 9- to 10-membered unsaturated heterocyclic group having 1 to 3 heteroatoms selected from the group consisting of N, S, and O.

The heterocyclic group is preferably a group having imidazole, pyrazole, thiophene, furan, pyrrole, oxazole, isoxazole, thiazole, isothiazole, triazole, tetrazole, pyridine, pyrazine, pyrimidine, pyridazine, indole, isoindole, pyrrolopyridine, indazole, methylenedioxyphenyl, ethylenedioxyphenyl, benzofuran, dihydrobenzofuran, benzimidazol, benzoxazol, benzothiazole, purine, quinoline, tetrahydroquinoline, isoquinoline, quinazoline, or quinoxaline, more preferably a group having imidazol, pyrazol, thiophene, furan, pyridine, indole, pyrrolopyridine, benzofuran, quinoline, or tetrahydroquinoline, and particularly preferably a group having imidazol, pyridine, or quinoline.

Specific examples thereof include a 1H-imidazol-1-yl group, a 1H-imidazol-2-yl group, a 1H-imidazol-4-yl group, a 1H-pyrazol-1-yl group, a 1H-pyrazol-3-yl group, a 1H-pyrazol-4-yl group, a thiophen-2-yl group, a thiophen-3-yl group, a furan-2-yl group, a furan-3-yl group, a pyrrol-1-yl group, a pyrrol-2-yl group, a pyrrol-3-yl group, an oxazol-2-yl group, an oxazol-4-yl group, an oxazol-5-yl group, an isoxazol-3-yl group, an isoxazol-4-yl group, an isoxazol-5-yl group, a thiazol-2-yl group, a thiazol-3-yl group, a thiazol-4-yl group, a thiazol-5-yl group, an isothiazol-2-yl group, an isothiazol-4-yl group, an isothiazol-5-yl group, a pyrazol-1-yl group, a pyrazol-3-yl group, a pyrazol-4-yl group, a 1,2,3-triazol-1-yl group, a 1,2,3-triazol-4-yl group, a 1,2,4-triazol-1-yl group, a 1,2,4-triazol-3-yl group, a 1,2,4-triazol-4-yl group, a tetrazol-1-yl group, a tetrazol-5-yl group, a pyridin-2-yl group, a pyridin-3-yl group, a pyridin-4-yl group, a pyrazin-2-yl group, a pyrazin-3-yl group, a pyrimidin-2-yl group, a pyrimidin-4-yl group, a pyrimidin-5-yl group, a pyrimidin-6-yl group, a pyridazin-3-yl group, a pyridazin-4-yl group, an indol-1-yl group, an indol-2-yl group, an indol-3-yl group, an indol-4-yl group, an indol-5-yl group, an indol-6-yl group, an indol-7-yl group, an isoindol-1-yl group, an isoindol-2-yl group, an isoindol-4-yl group, an isoindol-5-yl group, a 1H-pyrrolo[2,3-blpyridin-1-yl group, a 1H-pyrrolo[2,3-b]pyridin-2-yl group, a 1H-pyrrolo[2,3-b]pyridin-3-yl group, a 1H-pyrrolo[2,3-b]pyridin-4-yl group, a 1H-pyrrolo[2,3-b]pyridin-5-yl group, a 1H-pyrrolo[2,3-b]pyridin-6-yl group, a 1H-indazol-1-yl group, a 1H-indazol-3-yl group, a 1H-indazol-4-yl group, a 1H-indazol-5-yl group, a 1H-indazol-6-yl group, a 1H-indazol-7-yl group, a methylenedioxyphenyl group, an ethylenedioxyphenyl group, a benzofuran-2-yl group, a benzofuran-3-yl group, a benzofuran-4-yl group, a benzofuran-5-yl group, a benzofuran-6-yl group, a benzofuran-7-yl group, a 2,3-dihydrobenzofuran-2-yl group, a 2,3-dihydrobenzofuran-3-yl group, a benzimidazol-1-yl group, a benzimidazol-2-yl group, a benzimidazol-4-yl group, a benzimidazol-5-yl group, a benzoxazol-2-yl group, a benzoxazol-4-yl group, a benzoxazol-5-yl group, a benzothiazol-2-yl group, a benzothiazol-4-yl group, a benzothiazol-5-yl group, a purin-2-yl group, a purin-6-yl group, a purin-7-yl group, a purin-8-yl group, a quinolin-2-yl group, quinolin-3-yl group, a quinolin-4-yl group, quinolin-5-yl group, a quinolin-6-yl group, a quinolin-7-yl group, a quinolin-8-yl group, a 5,6,7,8-tetrahydroquinolin-2-yl group, a 5,6,7,8-tetrahydroquinolin-3-yl group, a 5,6,7,8-tetrahydroquinolin-4-yl group, an isoquinolin-1-yl group, an isoquinolin-3-yl group, an isoquinolin-4-yl group, an isoquinolin-5-yl group, an

isoquinolin-6-yl group, an isoquinolin-7-yl group, an isoquinolin-8-yl group, a quinazolin-4-yl group, a quinoxalin-2-yl group, a quinoxalin-5-yl group, and a quinoxalin-6-yl group. A 1H-imidazol-1-yl group, a pyrazol-4-yl group, a thiophen-3-yl group, a furan-2-yl group, a pyridin-3-yl group, a pyridin-4-yl group, an indol-5-yl group, a 1H-pyrrolo[2,3-b]pyridin-5-yl group, a benzofuran-2-yl group, a quinolin-3-yl group, and 5,6,7,8-tetrahydroquinolin-3-yl group are preferable, a 1H-imidazol-1-yl group, a pyridin-3-yl group, a pyridin-4-yl group, an indol-5-yl group, a 1H-pyrrolo[2,3-b]pyridin-5-yl group, a benzofuran-2-yl group, a quinolin-3-yl group, and a 5,6,7,8-tetrahydroquinolin-3-yl group are more preferable, and a 1H-imidazol-1-yl group, a pyridin-3-yl group, and a quinolin-3-yl group are particularly preferable.

In Formula (I), examples of the "substituent(s)" in the unsaturated heterocyclic group represented by $R^1$ include the substituents described above. The substituent(s) are preferably 1 to 3 substituents selected from the group consisting of an alkyl group, an alkoxy group, an alkoxy-alkyl group, an aralkyl group, an aralkyloxy-alkyl group, a halogen atom, a halogenoalkyl group, an acyl group, an optionally substituted saturated or unsaturated heterocyclic group, and an optionally substituted aromatic hydrocarbon group, more preferably 1 to 3 substituents selected from the group consisting of an alkyl group; an alkoxy group; an unsaturated heterocyclic group optionally having an alkyl group, a halogenoalkyl group, an aralkyl group, or a hydroxyalkyl group; and an aromatic hydrocarbon group optionally having an alkyl group, an alkoxy group, or a carbamoyl group. Herein, examples of the unsaturated heterocyclic group which may be substituted on the unsaturated heterocyclic ring represented by $R^1$ include pyrazol, imidazol, pyridine, pyrimidine, furan, and thiophene. In addition, examples of the aromatic hydrocarbon group include phenyl and naphthyl.

Specific examples of the "substituent(s)" in the unsaturated heterocyclic group represented by $R^1$ include a methyl group, an ethyl group, an n-propyl group, an isopropyl group, an n-butyl group, an isobutyl group, a sec-butyl group, a tert-butyl group, a methoxy group, an ethoxy group, an n-propoxy group, an isopropoxy group, a 1-methylpropoxy group, an n-butoxy group, an isobutoxy group, a tert-butoxy group, a 1H-pyrazol-4-yl group, a 1-methyl-1H-pyrazol-4-yl group, a 1-ethyl-1H-pyrazol-4-yl group, a 1-isopropyl-1H-pyrazol-4-yl group, a 1-benzyl-1H-pyrazol-4-yl group, a 1-(difluoromethyl)-1H-pyrazol-4-yl group, a 1-(hydroxyethyl)-1H-pyrazol-4-yl group, a 1H-imidazol-1-yl group, a pyridin-3-yl group, a pyridin-4-yl group, a pyrimidin-5-yl group, a furan-2-yl group, a furan-3-yl group, a thiophen-3-yl group, a phenyl group, a 4-methoxyphenyl group, a 4-carbamoylphenyl group, a 4-isopropylcarbamoylphenyl group, and a 4-dimethylcarbamoylphenyl group.

Specific examples of preferable $R^1$ include a 1H-imidazol-1-yl group, a 4-phenyl-1H-imidazol-1-yl group, a 4-(4-carbamoylphenyl)-1H-imidazol-1-yl group, a 4-(4-methoxyphenyl)-1H-imidazol-1-yl group, a 4-(thiophene-3-yl)-1H-imidazol-1-yl group, a 4-(pyridin-3-yl)-1H-imidazol-1-yl group, a 4-(pyridin-4-yl)-1H-imidazol-1-yl group, a 5-methyl-4-(pyridin-3-yl)-1H-imidazol-1-yl group, a 4-(pyrimidin-5-yl)-1H-imidazol-1-yl group, a 4-(furan-2-yl)-1H-imidazol-1-yl group, a 4-(furan-3-yl)-1H-imidazol-1-yl group, a 4-(1H-pyrazol-4-yl)-1H-imidazol-1-yl group, a 4-(1-methyl-1H-pyrazol-4-yl)-1H-imidazol-1-yl group, a 4-(1-ethyl-1H-pyrazol-4-yl)-1H-imidazol-1-yl group, a 4-(1-isopropyl-1H-pyrazol-4-yl)-1H-imidazol-1-yl group, a 4-(1-hydroxymethyl)-(1H-pyrazol-4-yl)-1H-imidazol-1-yl group, a 4-(1-(difluoromethyl)-1H-pyrazol-4-yl)-1H-imidazol-1-yl group, a 4-(1-(hydroxyethyl)-1H-pyrazol-4-yl)-1H-imidazol-1-yl group, a 4-(1-(hydroxymethyl)-1H-pyrazol-4-y1)-1H-imidazol-1-yl group, a 4-(1-benzyl-1H-pyrazol-4-yl)-1H-imidazol-1-yl group, a 4-(1-(benzyloxyethyl)-1H-pyrazol-4-yl)-1H-imidazol-1-yl group, a 1'H-1,4'-biimidazol-1'-yl group, a pyridin-3-yl group, a pyridin-4-yl group, a 5-methoxypyridin-3-yl group, a 6-methoxypyridin-3-yl group, a 1-benzyl-1H-pyrazol-4-yl group, a 1-methyl-1H-indol-5-yl group, a 1H-pyrrolo[2,3-b]pyridin-5-yl group, a 1-methyl-1H-pyrrolo[2,3-b]pyridin-5-yl group, a 1-methoxymethyl-1H-pyrrolo[2,3-b]pyridin-5-yl group, a 5,6,7,8-tetrahydroquinolin-3-yl group, a quinolin-3-yl group, a thiophen-3-yl group, a furan-2-yl group, and a benzofuran-2-yl group. $R^1$ is more preferably 1H-imidazol-1-yl group, a 4-(pyridin-3-yl)-1H-imidazol-1-yl group, a 4-(pyridin-4-yl)-1H-imidazol-1-yl group, a 4-(1H-pyrazol-4-yl)-1H-imidazol-1-yl group, a 4-(1-methyl-1H-pyrazol-4-yl)-1H-imidazol-1-yl group, a 4-(1-ethyl-1H-pyrazol-4-yl)-1H-imidazol-1-yl group, a 4-(1-isopropyl-1H-pyrazol-4-yl)-1H-imidazol-1-yl group, a 4-(1-benzyl-1H-pyrazol-4-yl)-1H-imidazol-1-yl group, or a quinolin-3-yl group, particularly preferably a 4-(1-methyl-1H-pyrazol-4-yl)-1H-imidazol-1-yl group, a 4-(pyridin-3-yl)-1H-imidazol-1-yl group, or a quinolin-3-yl group.

In Formula (I), the "alkyl group having 1 to 6 carbon atoms" in the "optionally substituted alkyl group having 1 to 6 carbon atoms" represented by $R^2$ refers to a linear or branched alkyl group having 1 to 6 carbon atoms, for example, a methyl group, an ethyl group, an n-propyl group, an isopropyl group, an n-butyl group, an isobutyl group, a sec-butyl group, a tert-butyl group, a pentyl group, or a hexyl group, and is preferably a methyl group, an ethyl group, an n-propyl group, or an isopropyl group.

Examples of the "substituent(s)" in the "optionally substituted alkyl group having 1 to 6 carbon atoms" represented by $R^2$ include the substituents described above. Among these substituents, halogen atoms are preferable.

The halogen atom-substituted alkyl group is preferably a halogenoalkyl group having 1 to 6 carbon atoms, more preferably a trifluoromethyl group.

The "alkenyl group having 2 to 6 carbon atoms" in the "optionally substituted alkenyl group having 2 to 6 carbon atoms" represented by $R^2$ refers to the alkenyl groups having 2 to 6 carbon atoms described above, and is preferably a vinyl group. Examples of the "substituent(s)" in the "optionally substituted alkenyl group having 2 to 6 carbon atoms" include the substituents described above.

$R^2$ is more preferably an optionally substituted alkyl group having 1 to 6 carbon atoms or an optionally substituted alkenyl group having 2 to 6 carbon atoms, even more preferably an alkyl group having 1 to 6 carbon atoms and optionally having a halogen atom, or an alkenyl group having 2 to 6 carbon atoms, particularly preferably an alkyl group having 1 to 4 carbon atoms and optionally having a halogen atom.

Any one or two of $Y^1$, $Y^2$, $Y^3$, and $Y^4$ are C-$R^4$, and the others are the same or different and represent CH or N. Preferably, any one or two of $Y^1$, $Y^2$, $Y^3$, and $Y^4$ are C-$R^4$, and the others are CH. More preferably, $Y^1$ and $Y^3$ are CH, any one or two of $Y^2$ and $Y^4$ are C-$R^4$, and the others are CH. These preferable aspects are represented by the following formulae:

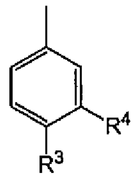
(b 1)

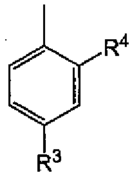
(b 2)

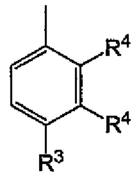
(b 3)

wherein, $R^3$ and $R^4$ are as defined above.

Among these, (b1) and (b2) are particularly preferable.

In Formula (I), $R^3$ represents a cyano group or -CO-$R^5$. Among these, -CO-$R^5$ is particularly preferable.

In Formula (I), $R^4$(s) are the same or different and represent a hydrogen atom, a halogen atom, a cyano group, an optionally substituted alkyl group having 1 to 6 carbon atoms, an alkenyl group having 2 to 6 carbon atoms, an alkoxy group having 1 to 6 carbon atoms, an aromatic hydrocarbon group, -N($R^6$)($R^7$), -S$R^8$, or - CO-$R^9$. Among these, $R^4$ is preferably a halogen atom, an alkyl group having 1 to 6 carbon atoms and optionally having a mono- or di-(C1-C6 alkyl)amino group or a monocyclic 5- to 7-membered saturated heterocyclic group having one or two heteroatoms of any of N, S, and O, an alkoxy group having 1 to 6 carbon atoms, -N($R^6$)($R^7$), -S-$R^8$, or -CO-$R^9$, more preferably a halogen atom, an alkyl group having 1 to 6 carbon atoms, or -N($R^6$)($R^7$).

In Formula (I), the "halogen atom" represented by $R^4$ refers to the halogen atom described above and is preferably a chlorine atom.

In Formula (I), the "alkyl group having 1 to 6 carbon atoms" in the "optionally substituted alkyl group having 1 to 6 carbon atoms" represented by $R^4$ refers to the alkyl group having 1 to 6 carbon atoms described above and is preferably a methyl group, an ethyl group, an n-propyl group, or an isopropyl group.

Examples of the "substituent(s)" in the "optionally substituted alkyl group having 1 to 6 carbon atoms" represented by $R^4$ include the substituents described above. The "substituent(s)" are preferably mono- or di-(C1-C6 alkyl)amino groups such as an ethylamino group and a dimethylamino group or monocyclic 5- to 7-membered saturated heterocyclic groups having one or two heteroatoms of any of N, S, and O such as a pyrrolidyl group and morpholino group.

In Formula (I), the "alkenyl group having 2 to 6 carbon atoms" represented by $R^4$ refers to the alkenyl group having 2 to 6 carbon atoms and is preferably a vinyl group or a prop-1-en-2-yl group.

In Formula (I), the "alkoxy group having 1 to 6 carbon atoms" represented by $R^4$ refers to the alkoxy group having 1 to 6 carbon atoms and is preferably a methoxy group.

In Formula (I), the "mono- or di-alkylamino group" in the "optionally substituted mono- or di-alkylamino group" represented by $R^5$ refers to the mono- or dialkylamino group described above, and is preferably a mono- or di-(C1-C6 alkyl)amino group.

Examples of the "substituent(s)" in the "optionally substituted mono- or di-alkylamino group" represented by $R^5$ include the substituents described above.

$R^5$ is more preferably an amino group, a hydroxylamino group, or a mono- or di-(C1-C6 alkyl)amino group, particularly preferably an amino group.

In Formula (I), the "alkyl group having 1 to 6 carbon atoms" in the "optionally substituted alkyl group having 1 to 6 carbon atoms" represented by $R^6$ or $R^7$ refers to the alkyl group having 1 to 6 carbon atoms described above, and is preferably an ethyl group, an n-propyl group, an n-butyl group, an isobutyl group, a sec-butyl group, or a pentyl group.

Examples of the "substituent(s)" in the "optionally substituted alkyl group having 1 to 6 carbon atoms" represented by $R^6$ or $R^7$ include the substituents described above. The "substituent(s)" are preferably a hydroxyl group, cycloalkyl groups having 3 to 7 carbon atoms (for example, a cyclohexyl group), saturated heterocyclic groups (for example, a pyrrolidyl group and a morpholino group), unsaturated heterocyclic groups (for example, a pyridyl group), mono- or di-(C1-C6 alkyl)amino groups (for example, an ethylamino group and a dimethylamino group), (C1-C6 alkyl)thio groups (for example, a methylthio group), or alkoxy groups having 1 to 6 carbon atoms and optionally having a hydroxyl

group.

In Formula (I), the "halogenoalkyl group having 1 to 6 carbon atoms" represented by $R^6$ or $R^7$ refers to the halogenoalkyl group having 1 to 6 carbon atoms described above, and is preferably a 2,2-difluoroethyl group or a 2,2,2-trifluoroethyl group.

In Formula (I), examples of the "cycloalkyl group having 3 to 7 carbon atoms" in the "optionally substituted cycloalkyl group having 3 to 7 carbon atoms" represented by $R^6$ or $R^7$ include a cyclopropyl group, a cyclobutyl group, a cyclopentyl group, a cyclohexyl group, and a cycloheptyl group, and is preferably a cyclopropyl group, a cyclopentyl group, or a cyclohexyl group.

Examples of the "substituent(s)" in the "optionally substituted cycloalkyl group having 3 to 7 carbon atoms" represented by $R^6$ or $R^7$ include the substituents described above. The substituent(s) are preferably a hydroxyl group, an amino group, an amino acid group-derived acyloxy group, an alkanoylamino group, or an alkylsulfonylamino group.

In Formula (I), the "aralkyl group" in the "optionally substituted aralkyl group" represented by $R^6$ or $R^7$ refers to the aralkyl group described above, and is preferably an aralkyl group having 7 to 12 carbon atoms, specifically, a benzyl group.

Examples of the "substituent(s)" in the "optionally substituted aralkyl group" represented by $R^6$ or $R^7$ include the substituents described above. Specific examples of the substituent(s) include saturated heterocyclic groups such as a pyrrolidinyl group.

In Formula (I), the "aromatic hydrocarbon group" in the "optionally substituted aromatic hydrocarbon group" represented by $R^6$ or $R^7$ refers to the aromatic hydrocarbon group having 6 to 14 of carbon atom described above, and is preferably a phenyl group.

Examples of the "substituent(s)" in the "optionally substituted aromatic hydrocarbon group" represented by $R^6$ or $R^7$ include the substituents described above. The substituent(s) are preferably halogen atoms, alkylthio groups (for example, a methylthio group), saturated heterocyclic groups (for example, a morpholino group), or substituted carbamoyl groups (for example, a pyrrolidine-carbonyl group).

In the Formula (I), the "saturated heterocyclic group" in the "optionally substituted saturated heterocyclic group" represented by $R^6$ or $R^7$ refers to the saturated heterocyclic group described above, and is preferably a piperidinyl group or a tetrahydropyranyl group.

Examples of the "substituent(s)" in the "optionally substituted saturated heterocyclic group" represented by $R^6$ or $R^7$ include the substituents described above. The substituent(s) are preferably alkyl groups having 1 to 6 carbon atoms (for example, a methyl group), acyl groups (for example, an acetyl group), carbonyl groups having a saturated heterocyclic group (for example, a 2,6-dihydroxypyrimidinyl-4-carbonyl group), or aminoalkylcarbonyl groups (for example, a 2-aminoacetyl group).

In Formula (I), the "unsaturated heterocyclic group" in the "optionally substituted unsaturated heterocyclic group" represented by $R^6$ or $R^7$ refers to the unsaturated heterocyclic group described above, and is preferably a pyridyl group or an oxazolyl group.

Examples of the "substituent(s)" in the "optionally substituted unsaturated heterocyclic group" represented by $R^6$ or $R^7$ include the substituents described above.

In Formula (I), the "saturated heterocyclic group" which is optionally formed by $R^6$ and $R^7$ together with the nitrogen atom to which they are attached refers to a mono- or bi-cyclic saturated heterocyclic group preferably having 1 to 4 atoms of any of oxygen, nitrogen, and sulfur, and for example, a pyrrolidinyl group, a piperidinyl group, a piperazinyl group, a hexamethyleneimino group, a morpholino group, a thiomorpholino group, a homopiperazinyl group, a tetrahydrofuranyl group, or tetrahydropyranyl group.

In Formula (I), it is preferred for the combination of $R^6$ and $R^7$ that $R^6$ be a hydrogen atom or an optionally substituted alkyl group having 1 to 6 carbon atoms; and $R^7$ represent a hydrogen atom, an optionally substituted alkyl group having 1 to 6 carbon atoms, an optionally substituted cycloalkyl group having 3 to 7 carbon atoms, an optionally substituted aralkyl group having 7 to 12 carbon atoms, an optionally substituted aromatic hydrocarbon group having 6 to 14 carbon atoms, an optionally substituted mono- or bi-cyclic saturated heterocyclic group having 1 to 4 heteroatoms selected from the group consisting of N, S, and O, or an optionally substituted mono- or bi-cyclic unsaturated heterocyclic group having 1 to 4 heteroatoms selected from the group consisting of N, S, and O, or $R^6$ and $R^7$ optionally form a 5- to 7-membered saturated heterocyclic group, together with the nitrogen atom to which they are attached. More preferably, $R^6$ is a hydrogen atom, and $R^7$ is a hydrogen atom, an optionally substituted alkyl group having 1 to 6 carbon atoms, an optionally substituted cycloalkyl group having 3 to 7 carbon atoms, or an optionally substituted mono- or bi-cyclic saturated heterocyclic group having 1 to 4 heteroatoms selected from the group consisting of N, S, and O. Particularly preferably, $R^6$ is a hydrogen atom, and $R^7$ is an optionally substituted alkyl group having 1 to 6 carbon atoms or an optionally substituted cycloalkyl group having 3 to 7 carbon atoms.

In Formula (I), the "cycloalkyl group having 3 to 7 carbon atoms" in the "optionally substituted cycloalkyl group having 3 to 7 carbon atoms" represented by $R^8$ refers to the cycloalkyl group having 3 to 7 carbon atoms described above, and is preferably a cyclohexyl group. Examples of the "substituent(s)" in the "optionally substituted cycloalkyl

group having 3 to 7 carbon atoms" represented by $R^8$ include the substituents described above. The substituent(s) are preferably a hydroxyl group.

In Formula (I), the "aromatic hydrocarbon group" in the "optionally substituted aromatic hydrocarbon group" represented by $R^8$ refers to the aromatic hydrocarbon group having 6 to 14 carbon atoms described above, and is preferably a phenyl group.

Examples of the "substituent(s)" in the "optionally substituted aromatic hydrocarbon group" represented by $R^8$ include the substituents described above. The substituent(s) are preferably a hydroxyl group.

$R^8$ is preferably an optionally substituted cycloalkyl group having 3 to 7 carbon atoms, or an optionally substituted aromatic hydrocarbon group having 6 to 14 carbon atoms.

In Formula (I), the "mono- or di-alkylamino group" in the "optionally substituted mono- or di-alkylamino group" represented by $R^9$ refers to the mono- or dialkylamino group described above, and is preferably a mono- or di-(C1-C6 alkyl)amino group.

Examples of the "substituent(s)" in the "optionally substituted mono- or di-alkylamino group" represented by $R^9$ include the substituents described above.

$R^9$ is preferably a hydrogen atom, a hydroxyl group, an amino group or a mono- or di-(C1-C6 alkyl)amino group, particularly preferably a hydrogen atom.

In the present invention, the preferred azabicyclo compound is a compound of Formula (I), wherein $X^1$ is CH or N; $X^2$ is N and $X^3$ and $X^4$ are CH; $Y^1$ and $Y^3$ are CH, any one or two of $Y^2$ and $Y^4$ are C-$R^4$, and the other is CH; $R^1$ is any of an optionally substituted 1H-imidazol-1-yl group, an optionally substituted pyrazol-4-yl group, an optionally substituted thiophen-3-yl group, an optionally substituted furan-2-yl group, an optionally substituted pyridin-3-yl group, an optionally substituted pyridin-4-yl group, an optionally substituted indol-5-yl group, an optionally substituted 1H-pyrrolo[2,3-b]pyridin-5-yl group, an optionally substituted benzofuran-2-yl group, an optionally substituted quinolin-3-yl group, and an optionally substituted 5,6,7,8-tetrahydroquinolin-3-yl group; $R^2$ is an alkyl group having 1 to 6 carbon atoms and optionally having a halogen atom or an alkenyl group having 2 to 6 carbon atoms; $R^3$ is -CO-$R^5$; $R^4$ is a halogen atom, an alkyl group having 1 to 6 carbon atoms and optionally having a mono- or di-(Cl-C6 alkyl)amino group or a monocyclic 5- to 7-membered saturated heterocyclic group having one or two heteroatoms of any of N, S, and O, an alkoxy group having 1 to 6 carbon atoms, -N($R^6$)($R^7$), -S-$R^8$, or -CO-$R^9$; $R^5$ is an amino group or mono- or di-(C1-C6 alkyl)amino group; $R^6$ is a hydrogen atom or an optionally substituted alkyl group having 1 to 6 carbon atoms; $R^7$ is a hydrogen atom, an optionally substituted alkyl group having 1 to 6 carbon atoms, an optionally substituted cycloalkyl group having 3 to 7 carbon atoms, an optionally substituted aralkyl group having 7 to 12 carbon atoms, an optionally substituted aromatic hydrocarbon group having 6 to 14 carbon atoms, an optionally substituted mono- or bi-cyclic saturated heterocyclic group having 1 to 4 heteroatoms selected from the group consisting of N, S, and O, or an optionally substituted mono- or bi-cyclic unsaturated heterocyclic group having 1 to 4 heteroatoms selected from the group consisting of N, S, and O, or $R^6$ and $R^7$ form a 5- to 7-membered saturated heterocyclic group together with a nitrogen atom to which they are attached; $R^8$ is an optionally substituted cycloalkyl group having 3 to 7 carbon atoms or an optionally substituted aromatic hydrocarbon group having 6 to 14 carbon atoms; and $R^9$ is a hydrogen atom, a hydroxyl group, an amino group, or a mono- or di-(C1-C6 alkyl)amino group.

More specifically, the azabicyclo compound is 3-ethyl-4-{3-isopropyl-4-(4-(1-methyl-1H-pyrazol-4-yl)-1H-imidazol-1-yl)-1H-pyrazolo[3,4-b]pyridin-1-yl}benzamide (hereinafter, referred to as Compound 1).

The HSP90-inhibiting compounds can be obtained as commercially available products, or produced by usual known methods. The azabicyclo compound of the above Formula (I) or a salt thereof can be synthesized according to the method described in WO 2011/004610 A, for example.

The salt of the HSP90-inhibiting compound of the present invention is not particularly limited as long as it is a pharmaceutically acceptable salt, and examples thereof include acid addition salts of inorganic acids (for example, hydrochloric acid, hydrobromic acid, hydroiodic acid, sulfuric acid, nitric acid, and phosphoric acid) and organic acids (for example, formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, p-toluenesulfonic acid, and glutamic acid); salts of inorganic bases (for example, sodium, potassium, magnesium, calcium, and aluminum), organic bases (for example, methylamine, ethylamine, meglumine, and ethanolamine), or a basic amino acids (for example, lysine, arginine, and ornithine); and ammonium salts.

The HSP90-inhibiting compound has inhibitory action on IDO expression as shown in Examples described below. Thus, the present invention relates to a prophylactic and/or therapeutic agent for diseases involving IDO expression, which has an HSP90-inhibiting compound as an active ingredient; an HSP90-inhibiting compound for preventing and/or treating diseases involving IDO expression; and a method for preventing and/or treating diseases involving IDO expression, the method including the step of administering an effective amount of an HSP90-inhibiting compound to a patient in need of prevention and/or treatment of a disease involving IDO expression. Examples of such diseases involving IDO expression include viral infections such as human immunodeficiency virus (HIV) infection, neurodegenerative diseases such as Alzheimer disease and Huntington disease, asthma, and autoimmune diseases such as rheumatoid

arthritis, multiple sclerosis, inflammatory bowel disease, psoriasis and systemic erythematosus.

The present invention includes a pharmaceutical composition for treating IDO-positive tumors, which has an HSP90-inhibiting compound as an active ingredient.

In the present invention, the "IDO-positive tumor" is a tumor in which an IDO protein or an IDO gene is detected, more preferably a tumor in which an IDO protein is detected.

The "treatment" includes postoperative adjuvant chemotherapy which is performed for prevention of recurrence after an IDO-positive tumor is removed surgically, and preoperative adjuvant chemotherapy which is preliminarily performed for surgically removing an IDO-positive tumor.

Examples of the method for detecting an IDO protein include usual common detection methods such as an ELISA method using an antibody which is attached specific to an IDO protein, a Western blotting method, and an immunostaining method. The antibody which is attached specific to an IDO protein can be obtained as a commercially available product, or produced by a usual common method.

In addition, examples of the method for detecting an IDO gene include usual common detection methods such as a Northern blotting method, a Southern blotting method, an RT-PCR method, a real time PCR method, a digital PCR method, a DNA microarray method, an in situ hybridization method, and a sequence analysis method.

In the present specification, "IDO expression" can be determined by a method for detecting an IOD protein and/or a method for detecting an IDO gene as described above.

Examples of the tumors included in IDO-positive tumors include malignant tumors, and specific examples thereof include head and neck cancer, digestive organ cancer (for example, esophageal cancer, stomach cancer, duodenal cancer, liver cancer, biliary tract cancer (for example, gallbladder/bile duct cancer), pancreatic cancer, small intestinal cancer, large intestine cancer (for example, colorectal cancer, colon cancer, or rectal cancer), or gastrointestinal stromal tumor), lung cancer (for example, non-small cell lung cancer or small cell lung cancer), breast cancer, ovarian cancer, uterus cancer (for example, cervical cancer or uterine corpus cancer), kidney cancer, bladder cancer, prostate cancer, skin cancer, testis tumor, bone/soft tissue sarcoma, leukemia, myelodysplastic syndrome, chronic myeloproliferative disease, malignant lymphoma, multiple myeloma, brain tumor, and mesothelioma. The tumor is preferably digestive organ cancer (for example, esophageal cancer, stomach cancer, duodenal cancer, liver cancer, biliary tract cancer (for example, gallbladder/bile duct cancer), pancreatic cancer, small intestinal cancer, large intestine cancer (for example, colorectal cancer, colon cancer, or rectal cancer), or gastrointestinal stromal tumor) or lung cancer (for example, non-small cell lung cancer or small cell lung cancer), more preferably stomach cancer, gastrointestinal stromal tumor or lung cancer (for example, non-small cell lung cancer or small cell lung cancer). Herein, the tumor includes not only primary tumor but also tumor metastasizing to other organ(s) (for example, liver).

In the present invention, the dosage of the HSP90-inhibiting compound or a salt thereof per administration day is preferably a recommended dosage.

In the present invention, the "recommended dosage", which is determined through a clinical trial or the like, is a dosage at which the maximum therapeutic effect is exhibited while safe use is assured without development of a serious side effect. Specific examples of the recommended dosage include dosages approved, recommended or suggested by public organizations such as Pharmaceuticals and Medical Devices Agency (PMDA), Food and Drug Administration (FDA) and European Medicines Agency (EMA), or corporations, and described in appended documents, interview forms, treatment guidelines or the like, and dosages approved by any of the public organizations which are PMDA, FDA and EMA are preferable.

The administration forms of the prophylactic and/or therapeutic agent for diseases involving IDO expression and the pharmaceutical composition for treating IDO-positive tumors according to the present invention are not particularly limited, and can be appropriately selected in accordance with the therapeutic purpose. Specific examples of the administration form include oral agents (for examples, tablets, coated tablets, powder, granules, capsules, and liquid), injections, suppositories, cataplasms, and ointments.

Oral agents are preferable for the azabicyclo compound of Formula (I), or a salt thereof and BIIB021. Injections are preferable for tanespimycin and ganetespib.

The prophylactic and/or therapeutic agent for diseases involving IDO expression and the pharmaceutical composition for treating IDO-positive tumors in the present invention can be prepared by a usual known method by use of a pharmaceutically acceptable carrier in accordance with the administration form. Such a carrier can be selected from the group consisting of a variety of carriers generally employed in pharmaceuticals, and examples thereof include an excipient, a binder, a disintegrant, a lubricant, a diluent, a solubilizing agent, a suspending agent, a tonicity agent, a pH-adjusting agent, a buffer, a stabilizer, a coloring agent, a flavoring agent, and a deodorant.

The present invention also includes a method for evaluating the effectiveness of chemotherapy with an HSP90-inhibiting compound, the method including the step of detecting an IDO protein or an IDO gene in a biological sample. The chemotherapy with an HSP90-inhibiting compound is evaluated as being effective when an IDO protein and/or an IDO gene are detected in a biological sample. Preferably, the chemotherapy with an HSP90-inhibiting compound is evaluated as being effective when an IDO protein is detected in a biological sample.

In the present invention, the "biological sample" includes not only samples (for example, cells, tissues, organs, body fluids (for example, blood and lymphatic fluid), digestive juice and urine) collected from a living body, but also nucleic acid extracts (for example, genome DNA extracts, mRNA extracts, and cDNA preparations or cRNA preparations prepared from mRNA extracts) and protein extracts obtained from the samples. In addition, the biological samples may be those subjected to formalin-fixation treatment, alcohol-fixation treatment, freezing treatment or paraffin-embedment treatment. Examples of the living body include humans and other mammals, for example, monkeys, mice, rats, rabbits, dogs, cats, bovines, horses, pigs, and sheep.

When the chemotherapy with an HSP90-inhibiting compound is intended for prevention and/or treatment of IDO-positive tumors, the biological sample is preferably a sample derived from a cancer patient, more preferably a sample containing tumor cells. In addition, the method for collecting a biological sample can be appropriately selected in accordance with the type of the biological sample.

Examples

Hereinafter, the present invention will be described in more detail by means of Examples, which should not be construed as limiting the invention. Many modifications can be made by a person having ordinary knowledge in the field of the invention without departing from the technical idea of the invention.

Example 1: Inhibitory effect of HSP90-inhibiting compound on IDO protein expression

Protein expression was detected by a Western blotting method. A human stomach cancer line NCI-N87 (CRL-5822) was purchased from American Type Culture Collection (ATCC). The cells were cultured in RPMI1640 (Wako Pure Chemical Industries, Ltd.) medium supplemented with 10% fetal bovine serum (FBS). The cells were seeded in a 6-well plate (#140675, Nunc) at $1 \times 10^6$ cells per well. The cells were cultured in a 5% $CO_2$ incubator at 37°C for 24 hours, INFγ (PeproTech, Inc.) at 100 U/mL and test substances [tanespimycin (Biotrend), ganetespib, BIIBO21 and Compound 1] in amounts of 0.001 μM, 0.01 μM, 0.1 μM and 1 μM each were then added, and the cells were further cultured for 24 hours. After the culturing, the cells were washed with ice-cooled PBS, and the cells were then lysed with a cytolytic agent. The cell lysate solution was centrifuged, and the soluble fraction was then removed, and subjected to thermal denaturation, followed by protein separation by SDS-PAGE.

10-fold dilution of TBST (Santa Cruz) with Milli-Q water was used as a washing solution, and the TBST solution containing 5% Blocking One (nacalai tesque) was used as an antibody diluting solution.

After completion of SDS-PAGE, the protein was transferred to a PVDF membrane (Trans-Blot Turbo Midi PVDF Transfer Pack, Biorad) by using a blotting device (Trans-Blot Turbo Transfer System, Biorad). The membrane after the transfer was blocked by incubation at room temperature for 60 minutes or more with Blocking One. The membrane after the transfer was immersed in 1000-fold dilution of IDO (D5J4E™) Rabbit mAb (Cell Signaling Technology) with the antibody diluting solution or 1000-fold dilution of GAPDH (14C10) Rabbit mAb (Cell Signaling Technology) with the antibody diluting solution, and subjected to overnight reaction. The PVDF membrane was washed once with the washing solution, and then subjected to reaction with a secondary antibody Anti-rabbit IgG, HRP-linked Antibody (Cell Signaling Technology), 1000-fold dilution with the antibody diluting solution. The PVDF membrane was washed three times with the washing solution, and then immersed in SuperSignal West Pico Chemiluminescent Substrate (Thermo Scientific) for detection of GAPDH, or SuperSignal West Dura Extended Duration Substrate (Thermo Scientific) for detection of IDO, and chemiluminescence was detected by using Image Analyzer Amersham Imager 600QC (GE Healthcare Japan Corporation).

Figure 1 shows the results.

Figure 1 shows that tanespimycin, ganetespib, BIIB021 and Compound 1 exhibited inhibitory effect on IDO expression in a concentration-dependent manner.

Example 2: Suppressive effect of HSP90-inhibiting compound on growth of IDO-positive cells

2-1. IDO expression in cell line to which IFNγ was added

Human stomach cancer line NCI-N87 (CRL-5822), human lung cancer line NCI-H1975 (CRL-5908) and human lung cancer line HCC827 (CRL-2868) were purchased from ATCC, and human gastrointestinal stromal tumor line GIST-T1 (GIST01) was purchased from Cosmo Bio Co., Ltd. NCI-N87, NCI-H1975 and HCC827 were cultured in RPM11640 (Wako Pure Chemical Industries, Ltd.) medium supplemented with 10% FBS, and GIST-T1 was cultured in DMEM (Wako Pure Chemical Industries, Ltd.) medium supplemented with 10% FBS. The IDO protein and GAPDH were detected in the same manner as in Example 1 described above.

Figure 2 shows the results.

Figure 2 shows that addition of IFNγ induced IDO expression in GIST-T1, HCC827, NCI-H1975 and NCI-N87.

2-2. Suppressive effect on growth in IDO-positive cells

The amount of ATP generated from surviving cells was determined by CellTiter-Glo2.0 Assay (#G9243, Promega Corporation) to evaluate the cell growth inhibition ability of test substance. The cells of GIST-T1, HCC827, NCI-H1975 and NCI-N87 were seeded in 96-well plates (#165305, Thermo Scientific) at $1 \times 10^4$ (NCI-N87), $2 \times 10^3$ (HCC827 and HCI-H1975) and $3 \times 10^3$ (GIST-T1) per well, and cultured in a 5% $CO_2$ incubator at 37°C for 24 hours. After the culturing for 24 hours, INFγ (PeproTech) at 100 U/mL and a test substance (Compound 1) in amounts of 0.001 μM, 0.003 μM, 0.01 μM, 0.03 μM, 0.1 μM, 0.3 μM, 1 μM, 3 μM and 10 μM each were added, and further cultured for 72 hours. Equivalent amount of CellTiter-Glo2.0 Assay reagent as the medium was added to each well, the mixture was stirred for 2 minutes using a shaker under shading condition, and the plate was then left to stand at room temperature for about 10 minutes. The intensity of luminescence was measured using a microplate reader (EnSpireTM Multimode Plate Reader, PerkinElmer Japan Co., Ltd.), and used as an index of the number of surviving cells in each well. Using the intensity of luminescence of a group not treated with a test substance (control group) as a control, the cell survival rate (%) was calculated by using the following equation. From the obtained survival rate, the concentration (IC50 (μM)), at which the number of surviving cells is reduced to 50% of the control, was determined by a curve fitting regression analysis tool using Regression Analysis Tool XLfit5.3.0.8 (IDBS Ltd.).

$$\text{Cell survival rate} = T/C \times 100$$

T: Amount of luminescence in wells with a test substance
C: Amount of luminescence in wells with no test substance

Table 1 and Figure 3 show the results.

[Table 1]

| Cell Line | IC50(μM) |
|---|---|
| GIST-T1 | 0.27 |
| HCC827 | 1.42 |
| NCI-H1975 | 0.81 |
| NCI-N87 | 0.38 |

Compound 1 exhibited suppressive effect on cell growth in a concentration-dependent manner against cells expressing IDO due to addition of IFNγ (IDO-positive cells).

The above results show that the HSP90-inhibiting compound exhibited an inhibitory effect on IDO expression and a suppressive effect on cell growth against IDO-positive cells.

Example 3: HSP90 expression in cell line to which IFNγ was added

NCI-N87 and NCI-H1975 were cultured in RPMI1650 (Wako Pure Chemical Industries, Ltd.) medium supplemented with 10% FBS. The tests were carried out in the same manner as in Example 1, except that, 1000-fold dilution of HSP90 (C45G5) Rabbit mAb (Cell Signaling Technology) with the antibody diluting solution was used as a primary antibody for detection of HSP90, and Anti-rabbit IgG, HRP-linked Antibody (Cell Signaling Technology) (Cell Signaling Technology) was used as a secondary antibody for detection of HSP90. In addition, detection of GAPDH was performed in the same manner as in Example 1.

Figure 4 shows the results.

Figure 4 shows that, in NCI-H1975 and NCI-N87, HSP90 expression was not changed by addition of IFNγ.

## Claims

1. A prophylactic and/or therapeutic agent for diseases involving IDO expression, comprising an HSP90-inhibiting compound as an active ingredient.

**2.** The prophylactic and/or therapeutic agent for diseases involving IDO expression according to claim 1, wherein the HSP90-inhibiting compound is at least one member selected from the group consisting of an azabicyclo compound of the following Formula (I), tanespimycin, ganetespib, BIIB021 and a salt thereof:

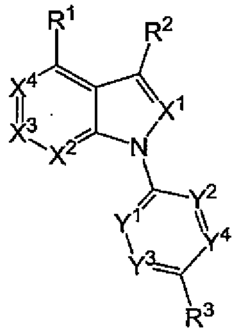

(I)

wherein, $X^1$ represents CH or N;
any one of $X^2$, $X^3$, and $X^4$ is N, and the others represent CH;
any one or two of $Y^1$, $Y^2$, $Y^3$, and $Y^4$ are C-$R^4$, and the others are the same or different and represent CH or N;
$R^1$ represents an optionally substituted mono- or bi-cyclic unsaturated heterocyclic group having 1 to 4 heteroatoms selected from the group consisting of N, S, and O;
$R^2$ represents a hydrogen atom, an optionally substituted alkyl group having 1 to 6 carbon atoms, or an optionally substituted alkenyl group having 2 to 6 carbon atoms;
$R^3$ represents a cyano group or -CO-$R^5$;
$R^4$(s) are the same or different and represent a hydrogen atom, a halogen atom, a cyano group, an optionally substituted alkyl group having 1 to 6 carbon atoms, an alkenyl group having 2 to 6 carbon atoms, an alkoxy group having 1 to 6 carbon atoms, an aromatic hydrocarbon group, -N($R^6$)($R^7$), -S-$R^8$, or -CO-$R^9$;
$R^5$ represents an amino group optionally having a hydroxyl group or an optionally substituted mono- or di-alkylamino group;
$R^6$ and $R^7$ are the same or different and represent a hydrogen atom, an optionally substituted alkyl group having 1 to 6 carbon atoms, a halogenoalkyl group having 1 to 6 carbon atoms, an optionally substituted cycloalkyl group having 3 to 7 carbon atoms, an optionally substituted aralkyl group, an optionally substituted aromatic hydrocarbon group, an optionally substituted saturated heterocyclic group, or an optionally substituted unsaturated heterocyclic group, or $R^6$ and $R^7$ optionally form a saturated heterocyclic group together with a nitrogen atom to which they are attached;
$R^8$ represents an optionally substituted cycloalkyl group having 3 to 7 carbon atoms or an optionally substituted aromatic hydrocarbon group; and
$R^9$ represents a hydrogen atom, a hydroxyl group, an amino group optionally having a hydroxyl group, or an optionally substituted mono- or di-alkylamino group.

**3.** The prophylactic and/or therapeutic agent for diseases involving IDO expression according to claim 2, wherein the azabicyclo compound is a compound of Formula (I),
wherein, $X^1$ is CH or N;
$X^2$ is N and $X^3$ and $X^4$ are CH;
$Y^1$ and $Y^3$ are CH, any one or two of $Y^2$ and $Y^4$ are C-$R^4$, and the other is CH;
$R^1$ is any of an optionally substituted 1H-imidazol-1-yl group, an optionally substituted pyrazol-4-yl group, an optionally substituted thiophen-3-yl group, an optionally substituted furan-2-yl group, an optionally substituted pyridin-3-yl group, an optionally substituted pyridin-4-yl group, an optionally substituted indol-5-yl group, an optionally substituted 1H-pyrrolo[2,3-b]pyridin-5-yl group, an optionally substituted benzofuran-2-yl group, an optionally substituted quinolin-3-yl group, and an optionally substituted 5,6,7,8-tetrahydroquinolin-3-yl group;
$R^2$ is an alkyl group having 1 to 6 carbon atoms and optionally having a halogen atom or an alkenyl group having 2 to 6 carbon atoms;
$R^3$ is -CO-$R^5$;
$R^4$ is a halogen atom, an alkyl group having 1 to 6 carbon atoms and optionally having a mono- or di-(C1-C6 alkyl)amino group or a monocyclic 5- to 7-membered saturated heterocyclic group having one or two heteroatoms of any of N, S, and O, an alkoxy group having 1 to 6 carbon atoms, -N($R^6$)($R^7$), -S-$R^8$, or -CO-$R^9$;
$R^5$ is an amino group or mono- or di-(C1-C6 alkyl)amino group;
$R^6$ is a hydrogen atom or an optionally substituted alkyl group having 1 to 6 carbon atoms;

$R^7$ is a hydrogen atom, an optionally substituted alkyl group having 1 to 6 carbon atoms, an optionally substituted cycloalkyl group having 3 to 7 carbon atoms, an optionally substituted aralkyl group having 7 to 12 carbon atoms, an optionally substituted aromatic hydrocarbon group having 6 to 14 carbon atoms, an optionally substituted mono- or bi-cyclic saturated heterocyclic group having 1 to 4 heteroatoms selected from the group consisting of N, S, and O, or an optionally substituted mono- or bi-cyclic unsaturated heterocyclic group having 1 to 4 heteroatoms selected from the group consisting of N, S, and O, or $R^6$ and $R^7$ form a 5- to 7-membered saturated heterocyclic group together with a nitrogen atom to which they are attached;
$R^8$ is an optionally substituted cycloalkyl group having 3 to 7 carbon atoms or an optionally substituted aromatic hydrocarbon group having 6 to 14 carbon atoms; and
$R^9$ is a hydrogen atom, a hydroxyl group, an amino group, or a mono- or di-(C1-C6 alkyl)amino group.

**4.** The prophylactic and/or therapeutic agent for diseases involving IDO expression according to claim 2 or 3, wherein the azabicyclo compound is 3-ethyl-4-{3-isopropyl-4-(4-(1-methyl-1H-pyrazol-4-yl)-1H-imidazol-1-yl)-1H-pyrazolo[3,4-b]pyridin-1-yl}benzamide.

**5.** A pharmaceutical composition for treating IDO-positive tumors, comprising an HSP90-inhibiting compound as an active ingredient.

**6.** An HSP90-inhibiting compound for use in preventing and/or treating diseases involving IDO expression.

**7.** The HSP90-inhibiting compound according to claim 6, wherein the HSP90-inhibiting compound is at least one member selected from the group consisting of an azabicyclo compound of the following Formula (I), tanespimycin, ganetespib, BIIB021 and a salt thereof:

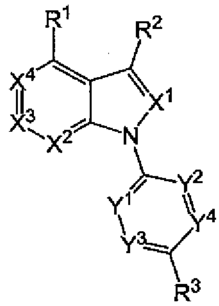

(I)

wherein, $X^1$ represents CH or N;
any one of $X^2$, $X^3$, and $X^4$ is N, and the others represent CH;
any one or two of $Y^1$, $Y^2$, $Y^3$, and $Y^4$ are C-$R^4$, and the others are the same or different and represent CH or N;
$R^1$ represents an optionally substituted mono- or bi-cyclic unsaturated heterocyclic group having 1 to 4 heteroatoms selected from the group consisting of N, S, and O;
$R^2$ represents a hydrogen atom, an optionally substituted alkyl group having 1 to 6 carbon atoms, or an optionally substituted alkenyl group having 2 to 6 carbon atoms;
$R^3$ represents a cyano group or -CO-$R^5$;
$R^4$(s) are the same or different and represent a hydrogen atom, a halogen atom, a cyano group, an optionally substituted alkyl group having 1 to 6 carbon atoms, an alkenyl group having 2 to 6 carbon atoms, an alkoxy group having 1 to 6 carbon atoms, an aromatic hydrocarbon group, -N($R^6$)($R^7$), -S-$R^8$, or -CO-$R^9$;
$R^5$ represents an amino group optionally having a hydroxyl group or an optionally substituted mono- or di-alkylamino group;
$R^6$ and $R^7$ are the same or different and represent a hydrogen atom, an optionally substituted alkyl group having 1 to 6 carbon atoms, a halogenoalkyl group having 1 to 6 carbon atoms, an optionally substituted cycloalkyl group having 3 to 7 carbon atoms, an optionally substituted aralkyl group, an optionally substituted aromatic hydrocarbon group, an optionally substituted saturated heterocyclic group, or an optionally substituted unsaturated heterocyclic group, or $R^6$ and $R^7$ optionally form a saturated heterocyclic group together with a nitrogen atom to which they are attached;
$R^8$ represents an optionally substituted cycloalkyl group having 3 to 7 carbon atoms or an optionally substituted aromatic hydrocarbon group; and
$R^9$ represents a hydrogen atom, a hydroxyl group, an amino group optionally having a hydroxyl group, or an optionally substituted mono- or di-alkylamino group.

**8.** The HSP90-inhibiting compound according to claim 7, wherein the azabicyclo compound is a compound of Formula (I),
wherein, $X^1$ is CH or N;
$X^2$ is N and $X^3$ and $X^4$ are CH;
$Y^1$ and $Y^3$ are CH, any one or two of $Y^2$ and $Y^4$ are C-$R^4$, and the other is CH;
$R^1$ is any of an optionally substituted 1H-imidazol-1-yl group, an optionally substituted pyrazol-4-yl group, an optionally substituted thiophen-3-yl group, an optionally substituted furan-2-yl group, an optionally substituted pyridin-3-yl group, an optionally substituted pyridin-4-yl group, an optionally substituted indol-5-yl group, an optionally substituted 1H-pyrrolo[2,3-b]pyridin-5-yl group, an optionally substituted benzofuran-2-yl group, an optionally substituted quinolin-3-yl group, and an optionally substituted 5,6,7,8-tetrahydroquinolin-3-yl group;
$R^2$ is an alkyl group having 1 to 6 carbon atoms and optionally having a halogen atom or an alkenyl group having 2 to 6 carbon atoms;
$R^3$ is -CO-$R^5$;
$R^4$ is a halogen atom, an alkyl group having 1 to 6 carbon atoms and optionally having a mono- or di-(C1-C6 alkyl)amino group or a monocyclic 5- to 7-membered saturated heterocyclic group having one or two heteroatoms of any one of N, S, and O, an alkoxy group having 1 to 6 carbon atoms, -N($R^6$)($R^7$), -S-$R^8$, or -CO-$R^9$;
$R^5$ is an amino group or mono- or di-(C1-C6 alkyl)amino group;
$R^6$ is a hydrogen atom or an optionally substituted alkyl group having 1 to 6 carbon atoms;
$R^7$ is a hydrogen atom, an optionally substituted alkyl group having 1 to 6 carbon atoms, an optionally substituted cycloalkyl group having 3 to 7 carbon atoms, an optionally substituted aralkyl group having 7 to 12 carbon atoms, an optionally substituted aromatic hydrocarbon group having 6 to 14 carbon atoms, an optionally substituted mono- or bi-cyclic saturated heterocyclic group having 1 to 4 heteroatoms selected from the group consisting of N, S, and O, or an optionally substituted mono- or bi-cyclic unsaturated heterocyclic group having 1 to 4 heteroatoms selected from the group consisting of N, S, and O, or $R^6$ and $R^7$ form a 5- to 7-membered saturated heterocyclic group together with a nitrogen atom to which they are attached;
$R^8$ is an optionally substituted cycloalkyl group having 3 to 7 carbon atoms or an optionally substituted aromatic hydrocarbon group having 6 to 14 carbon atoms; and
$R^9$ is a hydrogen atom, a hydroxyl group, an amino group, or a mono- or di-(C1-C6 alkyl)amino group.

**9.** The HSP90-inhibiting compound according to claim 7 or 8, wherein the azabicyclo compound is 3-ethyl-4-{3-isopropyl-4-(4-(1-methyl-1H-pyrazol-4-yl)-1H-imidazol-1-yl)-1H-pyrazolo[3,4-b]pyridin-1-y1}benzamide.

**10.** An HSP90-inhibiting compound which is used as a pharmaceutical composition for treating IDO-positive tumors.

**11.** Use of an HSP90-inhibiting compound for producing a prophylactic and/or therapeutic agent for diseases involving IDO expression.

**12.** The use according to claim 11, wherein the HSP90-inhibiting compound is at least one member selected from the group consisting of an azabicyclo compound of the following Formula (I), tanespimycin, ganetespib, BIIB021 and a salt thereof:

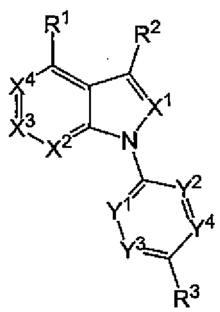

(I)

wherein, $X^1$ represents CH or N;
any one of $X^2$, $X^3$, and $X^4$ is N, and the others represent CH;
any one or two of $Y^1$, $Y^2$, $Y^3$, and $Y^4$ are C-$R^4$, and the others are the same or different and represent CH or N;
$R^1$ represents an optionally substituted mono- or bi-cyclic unsaturated heterocyclic group having 1 to 4 heteroatoms selected from the group consisting of N, S, and O;
$R^2$ represents a hydrogen atom, an optionally substituted alkyl group having 1 to 6 carbon atoms, or an optionally

substituted alkenyl group having 2 to 6 carbon atoms;
$R^3$ represents a cyano group or -CO-$R^5$;
$R^4$(s) are the same or different and represent a hydrogen atom, a halogen atom, a cyano group, an optionally substituted alkyl group having 1 to 6 carbon atoms, an alkenyl group having 2 to 6 carbon atoms, an alkoxy group having 1 to 6 carbon atoms, an aromatic hydrocarbon group, -N($R^6$)($R^7$), -S-$R^8$, or -CO-$R^9$;
$R^5$ represents an amino group optionally having a hydroxyl group or an optionally substituted mono- or di-alkylamino group;
$R^6$ and $R^7$ are the same or different and represent a hydrogen atom, an optionally substituted alkyl group having 1 to 6 carbon atoms, a halogenoalkyl group having 1 to 6 carbon atoms, an optionally substituted cycloalkyl group having 3 to 7 carbon atoms, an optionally substituted aralkyl group, an optionally substituted aromatic hydrocarbon group, an optionally substituted saturated heterocyclic group, or an optionally substituted unsaturated heterocyclic group, or $R^6$ and $R^7$ optionally form a saturated heterocyclic group together with a nitrogen atom to which they are attached;
$R^8$ represents an optionally substituted cycloalkyl group having 3 to 7 carbon atoms or an optionally substituted aromatic hydrocarbon group; and
$R^9$ represents a hydrogen atom, a hydroxyl group, an amino group optionally having a hydroxyl group, or an optionally substituted mono- or di-alkylamino group.

**13.** The use according to claim 12, wherein the azabicyclo compound is a compound of Formula (I),
wherein, $X^1$ is CH or N;
$X^2$ is N and $X^3$ and $X^4$ are CH;
$Y^1$ and $Y^3$ are CH, any one or two of $Y^2$ and $Y^4$ are C-$R^4$, and the other is CH;
$R^1$ is any of an optionally substituted 1H-imidazol-1-yl group, an optionally substituted pyrazol-4-yl group, an optionally substituted thiophen-3-yl group, an optionally substituted furan-2-yl group, an optionally substituted pyridin-3-yl group, an optionally substituted pyridin-4-yl group, an optionally substituted indol-5-yl group, an optionally substituted 1H-pyrrolo[2,3-b]pyridin-5-yl group, an optionally substituted benzofuran-2-yl group, an optionally substituted quinolin-3-yl group, and an optionally substituted 5,6,7,8-tetrahydroquinolin-3-yl group;
$R^2$ is an alkyl group having 1 to 6 carbon atoms and optionally having a halogen atom or an alkenyl group having 2 to 6 carbon atoms;
$R^3$ is -CO-$R^5$;
$R^4$ is a halogen atom, an alkyl group having 1 to 6 carbon atoms and optionally having a mono- or di-(C1-C6 alkyl)amino group or a monocyclic 5- to 7-membered saturated heterocyclic group having one or two heteroatoms of any of N, S, and O, an alkoxy group having 1 to 6 carbon atoms, -N($R^6$)($R^7$), -S-$R^8$, or -CO-$R^9$;
$R^5$ is an amino group or mono- or di-(C1-C6 alkyl)amino group;
$R^6$ is a hydrogen atom or an optionally substituted alkyl group having 1 to 6 carbon atoms;
$R^7$ is a hydrogen atom, an optionally substituted alkyl group having 1 to 6 carbon atoms, an optionally substituted cycloalkyl group having 3 to 7 carbon atoms, an optionally substituted aralkyl group having 7 to 12 carbon atoms, an optionally substituted aromatic hydrocarbon group having 6 to 14 carbon atoms, an optionally substituted mono- or bi-cyclic saturated heterocyclic group having 1 to 4 heteroatoms selected from the group consisting of N, S, and O, or an optionally substituted mono- or bi-cyclic unsaturated heterocyclic group having 1 to 4 heteroatoms selected from the group consisting of N, S, and O, or $R^6$ and $R^7$ form a 5- to 7-membered saturated heterocyclic group together with a nitrogen atom to which they are attached;
$R^8$ is an optionally substituted cycloalkyl group having 3 to 7 carbon atoms or an optionally substituted aromatic hydrocarbon group having 6 to 14 carbon atoms; and
$R^9$ is a hydrogen atom, a hydroxyl group, an amino group, or a mono- or di-(C1-C6 alkyl)amino group.

**14.** The use according to claim 12 or 13, wherein the azabicyclo compound is 3-ethyl-4-{3-isopropyl-4-(4-(1-methyl-1H-pyrazol-4-yl)-1H-imidazol-1-yl)-1H-pyrazolo[3,4-b]pyridin-1-yl}benzamide.

**15.** Use of an HSP90-inhibiting compound for producing a pharmaceutical composition for treating IDO-positive tumors.

**16.** A method for preventing and/or treating diseases involving IDO expression, the method comprising administering to a subject in need thereof an effective amount of an HSP90-inhibiting compound.

**17.** The method according to claim 16, wherein the HSP90-inhibiting compound is at least one member selected from the group consisting of an azabicyclo compound of the following Formula (I), tanespimycin, ganetespib, BIIB021 and a salt thereof:

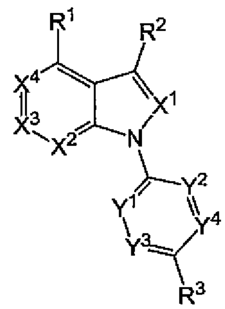

(I)

wherein, $X^1$ represents CH or N;
any one of $X^2$, $X^3$, and $X^4$ is N, and the others represent CH;
any one or two of $Y^1$, $Y^2$, $Y^3$, and $Y^4$ are C-$R^4$, and the others are the same or different and represent CH or N;
$R^1$ represents an optionally substituted mono- or bi-cyclic unsaturated heterocyclic group having 1 to 4 heteroatoms selected from the group consisting of N, S, and O;
$R^2$ represents a hydrogen atom, an optionally substituted alkyl group having 1 to 6 carbon atoms, or an optionally substituted alkenyl group having 2 to 6 carbon atoms;
$R^3$ represents a cyano group or -CO-$R^5$;
$R^4$(s) are the same or different and represent a hydrogen atom, a halogen atom, a cyano group, an optionally substituted alkyl group having 1 to 6 carbon atoms, an alkenyl group having 2 to 6 carbon atoms, an alkoxy group having 1 to 6 carbon atoms, an aromatic hydrocarbon group, -N($R^6$)($R^7$), -S-$R^8$, or -CO-$R^9$;
$R^5$ represents an amino group optionally having a hydroxyl group or an optionally substituted mono- or dialkylamino group;
$R^6$ and $R^7$ are the same or different and represent a hydrogen atom, an optionally substituted alkyl group having 1 to 6 carbon atoms, a halogenoalkyl group having 1 to 6 carbon atoms, an optionally substituted cycloalkyl group having 3 to 7 carbon atoms, an optionally substituted aralkyl group, an optionally substituted aromatic hydrocarbon group, an optionally substituted saturated heterocyclic group, or an optionally substituted unsaturated heterocyclic group, or $R^6$ and $R^7$ optionally form a saturated heterocyclic group together with a nitrogen atom to which they are attached;
$R^8$ represents an optionally substituted cycloalkyl group having 3 to 7 carbon atoms or an optionally substituted aromatic hydrocarbon group; and
$R^9$ represents a hydrogen atom, a hydroxyl group, an amino group optionally having a hydroxyl group, or an optionally substituted mono- or di-alkylamino group.

**18.** The method according to claim 17, wherein the azabicyclo compound is a compound of Formula (I),
wherein, $X^1$ is CH or N;
$X^2$ is N and $X^3$ and $X^4$ are CH;
$Y^1$ and $Y^2$ are CH, any one or two of $Y^2$ and $Y^4$ are C-$R^4$, and the other is CH;
$R^1$ is any of an optionally substituted 1H-imidazol-1-yl group, an optionally substituted pyrazol-4-yl group, an optionally substituted thiophen-3-yl group, an optionally substituted furan-2-yl group, an optionally substituted pyridin-3-yl group, an optionally substituted pyridin-4-yl group, an optionally substituted indol-5-yl group, an optionally substituted 1H-pyrrolo[2,3-b]pyridin-5-yl group, an optionally substituted benzofuran-2-yl group, an optionally substituted quinolin-3-yl group, and an optionally substituted 5,6,7,8-tetrahydroquinolin-3-yl group;
$R^2$ is an alkyl group having 1 to 6 carbon atoms and optionally having a halogen atom or an alkenyl group having 2 to 6 carbon atoms;
$R^3$ is -CO-$R^5$;
$R^4$ is a halogen atom, an alkyl group having 1 to 6 carbon atoms and optionally having a mono- or di-(C1-C6 alkyl)amino group or a monocyclic 5- to 7-membered saturated heterocyclic group having one or two heteroatoms of any of N, S, and O, an alkoxy group having 1 to 6 carbon atoms, -N($R^6$)($R^7$), -S-$R^8$, or -CO-$R^9$;
$R^5$ is an amino group or mono- or di-(C1-C6 alkyl)amino group;
$R^6$ is a hydrogen atom or an optionally substituted alkyl group having 1 to 6 carbon atoms;
$R^7$ is a hydrogen atom, an optionally substituted alkyl group having 1 to 6 carbon atoms, an optionally substituted cycloalkyl group having 3 to 7 carbon atoms, an optionally substituted aralkyl group having 7 to 12 carbon atoms, an optionally substituted aromatic hydrocarbon group having 6 to 14 carbon atoms, an optionally substituted mono- or bi-cyclic saturated heterocyclic group having 1 to 4 heteroatoms selected from the group consisting of N, S, and O, or an optionally substituted mono- or bi-cyclic unsaturated heterocyclic group having 1 to 4 heteroatoms selected from the group consisting of N, S, and O, or $R^6$ and $R^7$ form a 5- to 7-membered saturated heterocyclic group

together with a nitrogen atom to which they are attached;
$R^8$ is an optionally substituted cycloalkyl group having 3 to 7 carbon atoms or an optionally substituted aromatic hydrocarbon group having 6 to 14 carbon atoms; and
$R^9$ is a hydrogen atom, a hydroxyl group, an amino group, or a mono- or di-(C1-C6 alkyl)amino group.

**19.** The method according to claim 17 or 18, wherein the azabicyclo compound is 3-ethyl-4-{3-isopropyl-4-(4-(1-methyl-1H-pyrazol-4-yl)-1H-imidazol-1-yl)-1H-pyrazolo[3,4-b]pyridin-1-yl}benzamide.

**20.** A method for preventing and/or treating IDO-positive tumors, the method comprising administering to a subject in need thereof an effective amount of an HSP90-inhibiting compound.

Figure 1

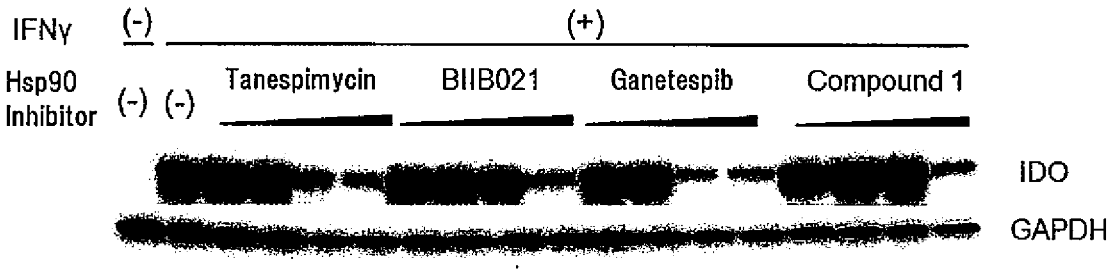


Figure 2

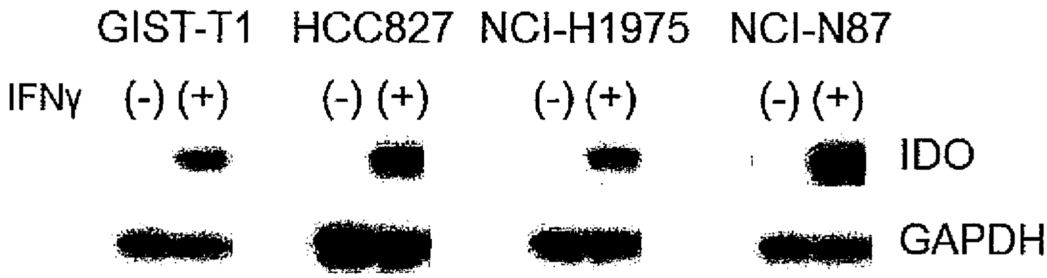

Figure 3

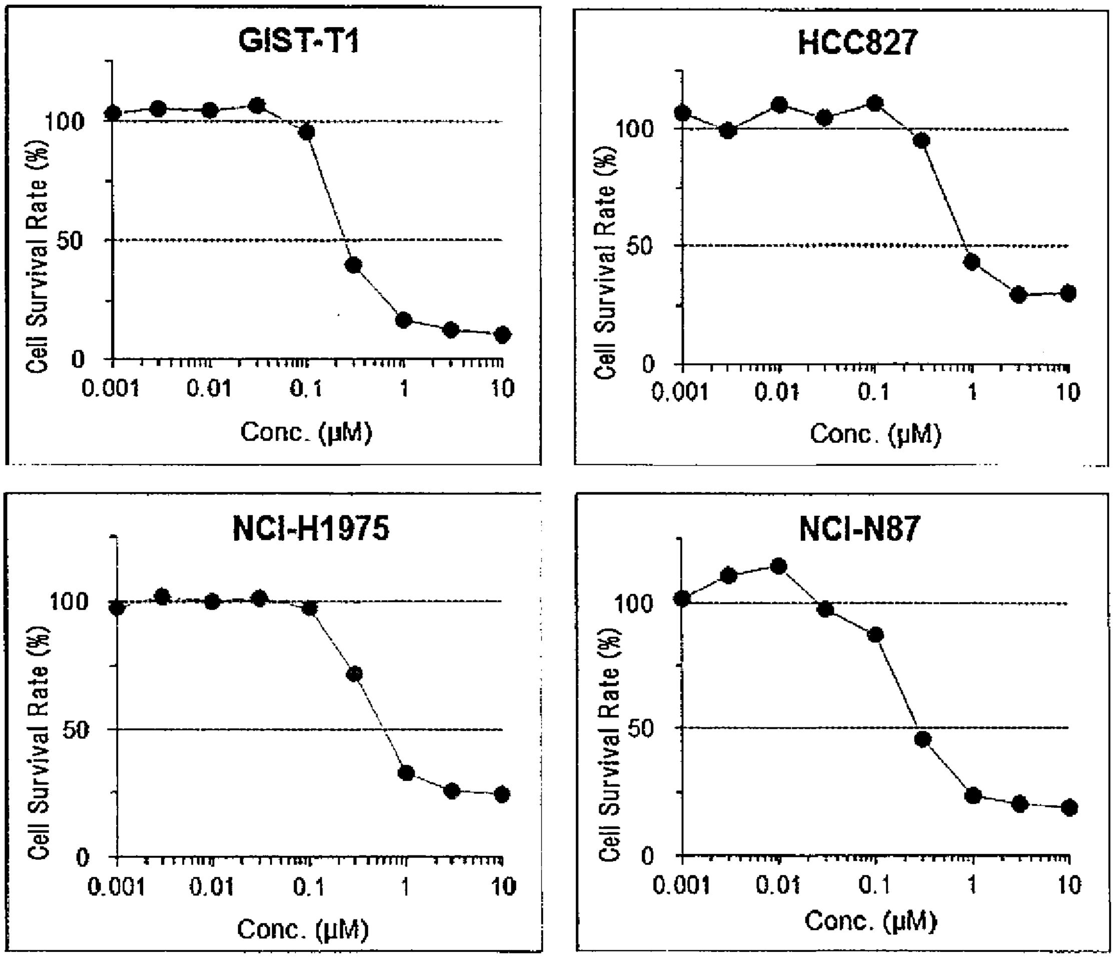


Figure 4

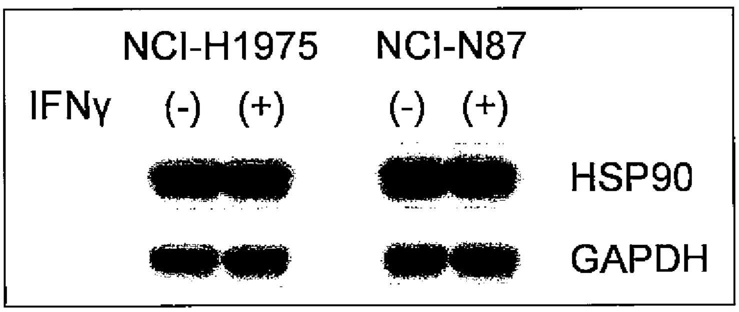

## INTERNATIONAL SEARCH REPORT

International application No.
PCT/JP2018/034092

A. CLASSIFICATION OF SUBJECT MATTER
Int.Cl. A61K45/00(2006.01)i, A61K31/395(2006.01)i, A61K31/4196(2006.01)i, A61K31/437(2006.01)i, A61K31/52(2006.01)i, A61P35/00(2006.01)i, A61P35/02(2006.01)i, A61P35/04(2006.01)i, A61P43/00(2006.01)i
According to International Patent Classification (IPC) or to both national classification and IPC

B. FIELDS SEARCHED
Minimum documentation searched (classification system followed by classification symbols)
Int.Cl. A61K45/00, A61K31/395, A61K31/4196, A61K31/437, A61K31/52, A61P35/00, A61P35/02, A61P35/04, A61P43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2011/004610 A1 (TAIHO PHARMACEUTICAL CO., LTD.) 13 January 2011 paragraphs [0001]-[0003], example 102, test example 2, table 1, claims & US 2012/0108589 A1, paragraphs [0001]-[0003], example 102, test example 2, table. 1, claims | 1-20<br>1-20 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 October 2018 (19.10.2018) | 06 November 2018 (06.11.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2018/034092

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2015-226536 A (MAX-PLANCK-GESELLSCHAFT ZUR FÖRDERUNG DER WISSENSCHAFTEN E.V.) 17 December 2015 claims, paragraph [0064] & WO 2010/020618 A1, claims, page 21 | 1-20<br>1-20 |
| X<br>Y | PROIA, D. A., et al., "Preclinical Activity Profile and Therapeutic Efficacy of the HSP90 Inhibitor Ganetespib in Triple-Negative Breast Cancer", Clin. Cancer Res., 2013, vol. 20, no. 2, pp. 413-424, abstract, DOI: 10.1158/1078-0432.CCR-13-2166 | 1-20<br>1-20 |
| X<br>Y | LIN, S., et al., "BIIB021, an Hsp90 inhibitor, effectively kills a myelodysplastic syndrome cell line via the activation of caspases and inhibition of PI3K/Akt and NF-κB pathway proteins", EXPERIMENTAL AND THERAPEUTIC MEDICINE, 2014, vol. 7, pp. 1539-1544, abstract, page 1539, right column, last paragraph to page 1540, DOI: 10.3892/etm.2014.1651 | 1-20<br>1-20 |
| A | WO 2015/046498 A1 (TAIHO PHARMACEUTICAL CO., LTD.) 02 April 2015 & US 2016/0228417 A1 | 1-20 |
| A | WO 2016/181990 A1 (TAIHO PHARMACEUTICAL CO., LTD.) 17 November 2016 & US 2018/0148443 A1 | 1-20 |

Form PCT/ISA/210 (continuation of second sheet) (January 2015)

**INTERNATIONAL SEARCH REPORT**

International application No.
PCT/JP2018/034092

Claims 1, 5, 6, 10, 11, 15, 16, and 20 pertain to an agent for preventing or treating cancer or the like, the agent having, as an active component, a compound defined by the desired property of being a "compound inhibiting HSP90".

Claims 1, 5, 6, 10, 11, 15, 16, and 20 encompass all compounds having this property. However, only a very small fraction of the claimed compounds are disclosed within the meaning of PCT Article 5. Thus, said claims are not considered to be supported by the description within the meaning of PCT Article 6.

Furthermore, with regard to the "compound inhibiting HSP90", the scope of compounds having such a property cannot be specified even in consideration of technical common knowledge at the date of filing. Thus, claims 1, 5, 6, 10, 11, 15, 16, 20 do not satisfy the requirement of clarity stipulated in PCT Article 6.

Therefore, a prior art document search was carried out by limiting the "compound inhibiting HSP90" to the aza bicyclic compound, tanespimycin, ganetespib, and BIIB021 tested in the examples in the present application and expressed by general formula (I) set forth in claim 2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011004610 A


### Non-patent literature cited in the description


- *Int. Immunopharmacol.,* 2017, vol. 47, 70-77
- *Nat. Rev. Cancer,* 2005, vol. 5, 761-772
- *TRENDS Mol. Med.,* 2004, vol. 10, 283-290
- *Clin. Cancer Res.,* 2009, vol. 15, 9-14
- *Curr. Opin. Pharmacol.,* 2008, vol. 8, 370-374
- *Drug Resist. Updat.,* 2009, vol. 12, 17-27